(19) Europäisches Patentamt
European Patent Office
Office européen des brevets

(11) EP 2 393 106 A1

(12) **EUROPEAN PATENT APPLICATION**
published in accordance with Art. 153(4) EPC

(43) Date of publication:
**07.12.2011 Bulletin 2011/49**

(21) Application number: **10735786.5**

(22) Date of filing: **26.01.2010**

(51) Int Cl.:
***H01L 21/027*** *(2006.01)* ***B29C 59/02*** *(2006.01)*
***C07C 275/10*** *(2006.01)* ***G11B 5/65*** *(2006.01)*
***G11B 5/84*** *(2006.01)* ***G11B 5/855*** *(2006.01)*
***B29K 61/20*** *(2006.01)*

(86) International application number:
**PCT/JP2010/050936**

(87) International publication number:
**WO 2010/087318 (05.08.2010 Gazette 2010/31)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.01.2009 JP 2009018384**

(71) Applicant: **Showa Denko K.K.**
**Tokyo 105-8518 (JP)**

(72) Inventors:
- **ARAI, Yoshikazu**
  **Tokyo 105-8518 (JP)**
- **UCHIDA, Hiroshi**
  **Tokyo 105-8518 (JP)**

(74) Representative: **Strehl Schübel-Hopf & Partner**
**Maximilianstrasse 54**
**80538 München (DE)**

(54) **CURABLE COMPOSITION FOR TRANSFER MATERIAL AND UREA COMPOUND CONTAINING (METH)ACRYLOYL GROUP**

(57) The invention provides curable compositions for transfer materials suited for nanoimprinting, which have very high transfer properties in a nanoimprinting process, show high selectivity in dry etching rate between by argon gas and by oxygen gas, and can be fabricated into fine patterns with high throughput. The curable composition for transfer materials includes a compound (A) having at least one skeleton selected from Formulae (1) to (3) below, and a (meth) acryloyl group:

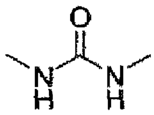

(1)

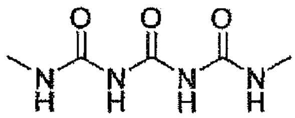

(2)

EP 2 393 106 A1

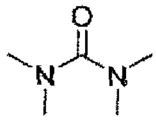

(3)

**Description**

TECHNICAL FIELD

The present invention relates to a curable composition for transfer materials, a pattern forming process using the composition, a process for manufacturing magnetic recording media using the pattern forming process, a magnetic recording medium obtainable by the process, a magnetic recording and reading apparatus having the medium, and a (meth)acryloyl group-containing urea compound.

BACKGROUND ART

Nanoimprinting technology attracts attention as a technique capable of forming fine patterns in the manufacturing processes for semiconductors or magnetic recording media such as patterned media. There has thus been a demand for excellent transfer materials for the use therein.

Thermoplastic resins such as polymethyl methacrylate are commonly used as nanoimprinting transfer materials. In detail, conventional cycles include heating the material that has been applied to at least the glass transition temperature thereof, pressing it with a mold, cooling, and separating the mold. However, such processes are time-consuming and have low throughput.

These problems are addressed by a technique in which a hydrogenated silsesquioxane that is a siloxane compound is applied on a substrate and the resultant layer is pressed with a mold at room temperature to fabricate a fine pattern (Patent Literature 1). A technique has been also disclosed in which a composition containing a catechol derivative and a resorcinol derivative is applied on a substrate and the resultant layer is pressed with a mold at room temperature to create a fine pattern (Patent Literature 2).

These processes are referred to as the room temperature imprinting processes and can be made without heating and cooling cycles. However, the mold pressing requires a long time and the throughput is still insufficient. Further, the high pressure applied for pressing shortens the life of stampers. Thus, these processes are not suited for mass production.

UV nanoimprinting technology has been then proposed which uses photocurable resins that can be cured with UV rays. In this process, a photocurable resin is applied and is cured by UV irradiation while being pressed with a stamper, and the stamper is thereafter separated to create a fine pattern. No heating and cooling cycles are necessary in this process. Further, the UV curing can be completed in a very short time, and the mold pressing can be made at low pressure. Thus, the process increases the possibility of solving the various problems described above.

The resins generally used in the UV nanoimprinting are acrylic organic resins. In the case of using the fine patterns produced as resists, the selectivity of etching rate with a variety of dry etching gases is important. Here, the selectivity of etching rate means that the etching rate varies depending on the kind of the etching gas. That the resin has a high selectivity of etching rate means that the etching rate is greatly different depending on the variety of the etching gases.

When a fine pattern functions as a resist, the resin should be highly resistant to the etching gas but should also permit easy removal when it is removed. That is, high selectivity of etching rate is required. Frequent etching gases are fluorine-containing gases and oxygen gas. In general, organic resins do not show a substantial difference in etching rate between by a fluorine-containing gas and by oxygen gas.

Then, silicon compounds are usually used in order to obtain the selectivity of etching rate between by a fluorine-containing gas and by oxygen gas. One example is the use of hydrogenated silsesquioxanes described above. They have a high etching rate by fluorine-containing gases while the etching rate thereof by oxygen gas is very low. However, the hydrogenated silsesquioxanes do not have photocurability and thus cannot be used in the UV nanoimprinting processes.

To solve such problems, a technique has been proposed which uses silicon compounds with a curable functional group that are synthesized by a sol-gel process (Patent Literature 3). According to this technique, however, increasing the molecular weight of the silicon compound during the sol-gel process results in gelation and the compound becomes insoluble in solvents and infusible. Therefore, the molecular weight cannot be increased. This process thus has problems that it is difficult to balance the strength and flexibility of fine patterns during and after the imprint formation.

Further, the use of fluorine-containing gases is sometimes avoided because of problems such as corrosion. A possible alternative is argon gas. Such cases thus require high selectivity of etching rate between by oxygen gas and by argon gas. The aforementioned silicon compounds are not etched easily under both of these conditions and are therefore not suited for use in these cases. There has been accordingly a need for compounds that show high etching resistance against argon gas and can be easily removed by etching with oxygen gas.

Patent Literature 4 discloses that compounds having a urea bond are used as thermal crosslinking agents in highly heat resistant photosensitive resin compositions for use as, for example, insulating materials in electronic parts.

The compounds having a urea bond allow for the photosensitive resins to show heat resistance and chemical resistance. However, Patent Literature 4 does not describe that the materials are used as imprinting resist materials.

Citation List

Patent Literatures

Patent Literature 1: JP-A-2003-100609
Patent Literature 2: JP-A-2005-277280
Patent Literature 3: JP-A-2007-72374
Patent Literature 4: JP-A-2003-287889

SUMMARY OF INVENTION

Technical Problem

It is an object of the invention to provide heat curable or energy ray curable compositions for transfer materials that have high selectivity in dry etching rate between by argon gas and by oxygen gas and can be fabricated into fine patterns used as resists or the like by a thermal or UV nanoimprinting process. It is another object of the invention to provide pattern-forming processes using the curable composition for transfer materials.

Solution to Problem

The present inventors studied diligently to solve the problems described hereinabove. They have then found that the use of a compound having a urea bond and a (meth) acryloyl group as an imprinting transfer material results in a composition that has very high imprinting properties and can give a cured pattern as a resist showing excellent etching resistance in the underlayer processing. The composition has been found to show excellent properties in the processing of semiconductors and magnetic recording media.

The present invention has embodiments as described in [1] to [16] below.

[1] A curable composition for transfer materials which comprises a compound (A) having at least one skeleton selected from the group consisting of Formulae (1) to (3) below, and a (meth)acryloyl group:

[Chem. 1]

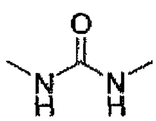

(1)

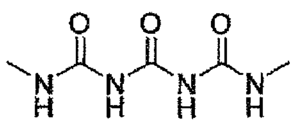

(2)

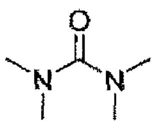

(3)

[2] The curable composition for transfer materials described in [1], wherein the compound (A) has 1 to 3 skeletons of Formula (1) in the molecule.

[3] The curable composition for transfer materials described in [1] or [2], wherein the compound (A) is at least one (meth)acryloyl group-containing urea compound selected from the group consisting of Formulae (4) and (5) below:

[Chem. 2]

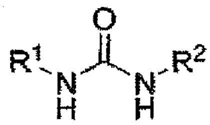

(4)

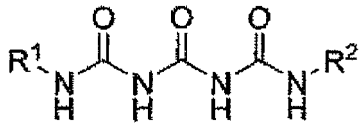

(5)

wherein $R^1$ and $R^2$ in Formulae (4) and (5) are each independently represented by any of Formulae (i) to (iii) below:

[Chem. 3]

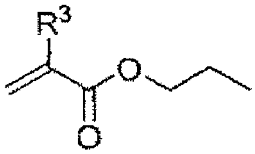

(i)

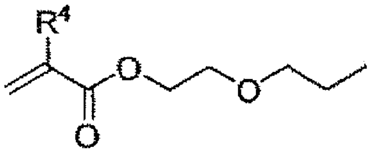

(ii)

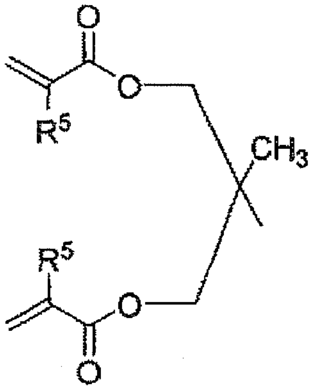

(iii)

wherein $R^3$ to $R^5$ in Formulae (i) to (iii) are each independently a hydrogen atom or a methyl group.

[4] The curable composition for transfer materials described in any one of [1] to [3], wherein the compound (A) is a compound obtained by reacting water and at least one (meth)acryloyl group-containing isocyanate selected from the group consisting of Formulae (iv) to (vi) below:

[Chem. 4]

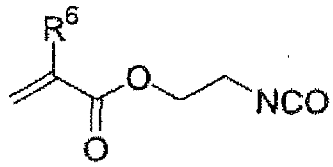

(iv)

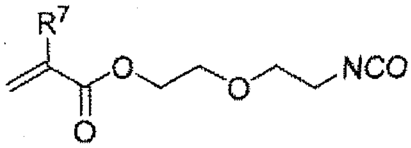

(v)

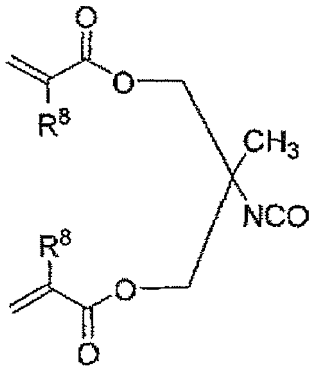

(vi)

wherein $R^6$, $R^7$ and $R^8$ in Formulae (iv) to (vi) are each independently a hydrogen atom or a methyl group.

[5] The curable composition for transfer materials described in any one of [1] to [3], wherein the compound (A) is a compound obtained by reacting urea and at least one (meth)acryloyl group-containing isocyanate selected from the group consisting of Formulae (iv) to (vi) below:

[Chem. 5]

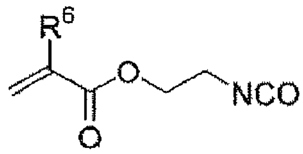

(iv)

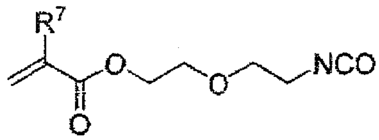

(v)

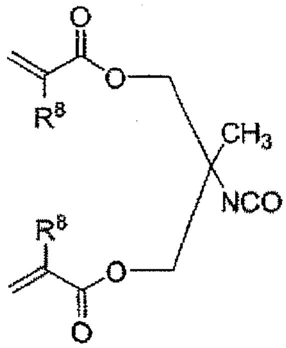

(vi)

wherein $R^6$, $R^7$ and $R^8$ in Formulae (iv) to (vi) are each independently a hydrogen atom or a methyl group.

[6] The curable composition for transfer materials described in [1] or [2], wherein the compound (A) is a compound obtained by reacting urea, a compound of Formula (vii) below having a hydroxyl group and a (meth)acryloyl group, and paraformaldehyde;

[Chem. 6]

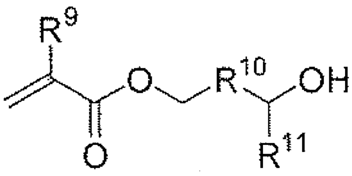

(vii)

wherein in Formula (vii), $R^9$ is a hydrogen atom or a methyl group, $R^{10}$ is a single bond or a C1-6 saturated hydrocarbon group, and $R^{11}$ is a hydrogen atom or a methyl group.

[7] The curable composition for transfer materials described in [6], wherein the compound of Formula (vii) is at least one selected from 2-hydroxyethyl (meth)acrylate, 2-hydroxypropyl (meth)acrylate, 3-hydroxybutyl (meth)acrylate, 4-hydroxybutyl (meth)acrylate and 1,4-cyclohexanedimethanol mono(meth)acrylate.

[8] The curable composition for transfer materials described in any one of [1] to [7], which further comprises a radically polymerizable compound other than the compound (A) .

[9] A pattern forming process comprising:

a step of applying the curable composition for transfer materials described in any one of [1] to [8] to a substrate to form a coating layer;
a step of pressing a mold having a pattern of projections and depressions on its surface, into the coating layer;
a step of heating the coating layer while keeping the mold pressed in the coating layer, thereby to cure the coating layer; and
a step of separating the mold from the cured coating layer.

[10] A pattern forming process comprising:

a step of applying the curable composition for transfer materials described in any one of [1] to [8] to a substrate to form a coating layer;
a step of pressing a mold having a pattern of projections and depressions on its surface, into the coating layer;
a step of applying an active energy ray to the coating layer while keeping the mold pressed in the coating layer, thereby to cure the coating layer; and
a step of separating the mold from the cured coating layer.

[11] The pattern forming process described in [10], wherein the mold is formed of a material that transmits active energy rays, and the active energy ray is applied to the coating layer through the mold.

[12] The pattern forming process described in [10], wherein the substrate is formed of a material that transmits active energy rays, and the active energy ray is applied to the coating layer through the substrate.

[13] A process for manufacturing magnetic recording media, comprising:

providing a substrate comprising a base and a magnetic layer thereon and forming a pattern on the magnetic layer by the process described in any one of [9] to [12]; and
removing a part of the magnetic layer or demagnetizing a part of the magnetic layer using the pattern as a resist.

[14] A magnetic recording medium obtainable by the process described in [13].

[15] A magnetic recording and reading apparatus comprising the magnetic recording medium described in [14].

[16] A (meth)acryloyl group-containing urea compound represented by Formula (6) below:

[Chem. 7]

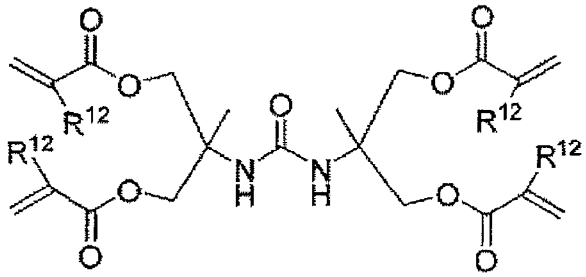

(6)

wherein $R^{12}$ in Formula (6) is a hydrogen atom or a methyl group.

Advantageous Effects of Invention

The curable compositions for transfer materials according to the invention can produce, with excellent transfer properties and high throughput, fine patterns having high selectivity of etching rate between by argon gas and by oxygen gas.

The pattern forming processes involving the curable composition for transfer materials according to the invention, and the patterns formed by the processes have the following three characteristics.

(1) Good transfer performance at the imprinting is achieved.
(2) The cured layer (pattern) formed from the composition may be easily removed by reactive ion etching using oxygen gas when the bottom of the imprinted layer is removed and perforated to expose the magnetic layer or the cured layer is removed.
(3) In the processing of media, the cured layer (pattern) as a resist shows good resistance to etching by argon gas.

Further, fine patterns in the production processes for semiconductors, magnetic recording media and the like may be fabricated by the pattern forming process of the invention.

BRIEF DESCRIPTION OF DRAWINGS

Fig. 1 shows steps in a pattern forming process using a curable composition for transfer materials according to the invention.
Fig. 2 shows an example of a quartz glass circular plate (diameter: 1.8 inch) used in Examples 6 to 10 and Comparative Example 2 in which projections and depressions are patterned along the radial direction. The width (L) of the depressions is 80 nm in the radial direction in Fig. 1, and the width (S) of the projections is 120 nm in the radial direction in the figure. The depth of the depressions is 150 nm. In an illustration shown on the right side of the glass circular plate in Fig. 2, the length in the vertical (lateral) direction of the rectangles corresponding to the mold is 0.1 mm.
Fig. 3 shows a perforation step in the processing of a magnetic layer in a magnetic recording medium.
Fig. 4 schematically shows partial removal or partial demagnetization of a magnetic layer in a magnetic recording medium.
Fig. 5 is a $^{1}$H-NMR spectrum of white solid powder (c) obtained in Example 3.
Fig. 6 is a $^{13}$C-NMR spectrum of the white solid powder (c) obtained in Example 3.
Fig. 7 is a field emission electron micrograph of a cross section exposed by cutting a glass substrate and a thin layer to which a pattern is transferred in Example 6.
Fig. 8 is a field emission electron micrograph of a cross section exposed by cutting a glass substrate and a thin layer to which a pattern is transferred in Example 7.
Fig. 9 is a field emission electron micrograph of a cross section exposed by cutting a glass substrate and a thin layer to which a pattern is transferred in Example 8.
Fig. 10 is a field emission electron micrograph of a cross section exposed by cutting a glass substrate and a thin layer to which a pattern is transferred in Example 9.
Fig. 11 is a field emission electron micrograph of a cross section exposed by cutting a glass substrate and a thin layer to which a pattern is transferred in Example 10.
Fig. 12 is a field emission electron micrograph of a cross section exposed by cutting a glass substrate and a thin layer to which a pattern is transferred in Comparative Example 2.

DESCRIPTION OF EMBODIMENTS

The present invention will be described in detail hereinbelow.

[Curable compositions for transfer materials]

A curable composition for transfer materials (hereinafter, also the curable composition) according to the invention includes a compound (A) having at least one skeleton selected from Formulae (1) to (3) below and a (meth)acryloyl group. In the invention, the (meth)acryloyl group indicates at least one selected from the acryloyl group and the methacryloyl group.

In the invention, the compounds (A) may be used singly, or two or more kinds may be used.
The compound (A) may be a compound that has 1 to 3 skeletons of Formula (1) in the molecule. If the compound (A) has 4 or more skeletons of Formula (1), the solubility is deteriorated and handling the compound is difficult. Thus, the number of the skeletons of Formula (1) is desirably from 1 to 3.

[Chem. 8]

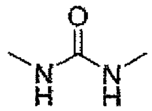

(1)

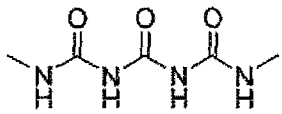

(2)

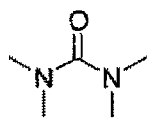

(3)

The compound (A) having the skeleton of Formula (1) (urea bond-containing compound) may be produced by, for example, reacting with water at least one (meth)acryloyl group-containing isocyanate selected from Formulae (iv) to (vi) described below.

The reaction between water and the at least one (meth)acryloyl group-containing isocyanate selected from Formulae (iv) to (vi) probably proceeds as follows. The at least one (meth) acryloyl group-containing isocyanate selected from Formulae (iv) to (vi) is hydrolyzed to a (meth)acryloyl group-containing amine compound, and the (meth)acryloyl group-containing amine compound is reacted with the at least one (meth)acryloyl group-containing isocyanate selected from Formulae (iv) to (vi) that has not been hydrolyzed to afford the compound (A) having the skeleton of Formula (1).

That is, the compound (A) may be a compound obtained by reacting water and at least one (meth)acryloyl group-containing isocyanate selected from Formulae (iv) to (vi) below.

[Chem. 9]

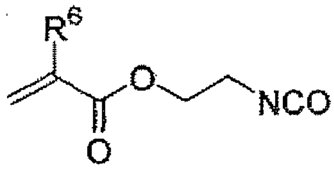

(iv)

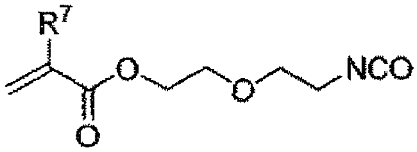

(v)

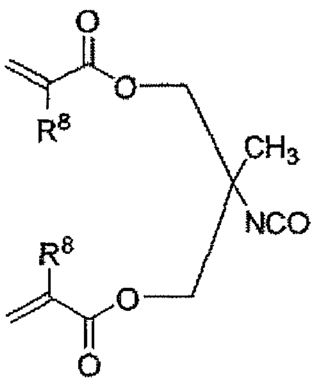

(vi)

In Formulae (iv) to (vi), $R^6$, $R^7$ and $R^8$ are each independently a hydrogen atom or a methyl group.
The compound resulting from the reaction between water and the (meth)acryloyl group-containing isocyanate is a (meth) acryloyl group-containing urea compound represented by Formula (4) described later.

Further, the compound (A) having the skeleton of Formula (2) (urea bond-containing compound) may be produced by, for example, reacting with urea at least one (meth)acryloyl group-containing isocyanate selected from Formulae (iv) to (vi) described above.

That is, the compound (A) may be a compound obtained by reacting urea and at least one (meth) acryloyl group-containing isocyanate selected from Formulae (iv) to (vi) described above.

The compound resulting from this reaction is a (meth)acryloyl group-containing urea compound represented by Formula (5) below.
That is, the compound (A) is preferably at least one (meth)acryloyl group-containing urea compound selected from Formulae (4) and (5).

[Chem. 10]

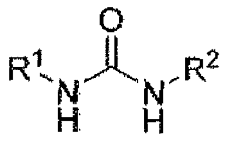

(4)

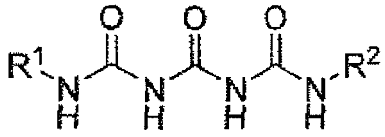

(5)

In Formulae (4) and (5), $R^1$ and $R^2$ are each independently represented by any of Formulae (i) to (iii) below:

[Chem. 11]

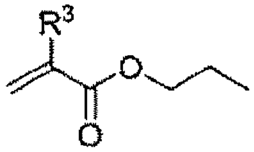

(i)

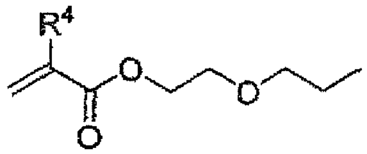

(ii)

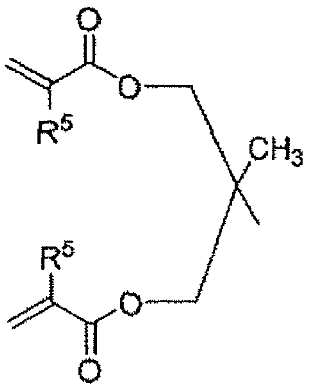

(iii)

In Formulae (i) to (iii), $R^3$ to $R^5$ are each independently a hydrogen atom or a methyl group.

Furthermore, the compound (A) having the skeleton of Formula (3) may be produced by, for example, reacting urea, a compound (B) having a hydroxyl group and a (meth)acryloyl group, and paraformaldehyde. Examples of the compounds (B) having a hydroxyl group and a (meth)acryloyl group include hydroxyalkyl (meth)acrylates such as 2-hydroxyethyl (meth)acrylate, 2-hydroxypropyl (meth)acrylate, 2-hydroxybutyl (meth)acrylate, 3-hydroxybutyl (meth)acrylate, 4-hydroxybutyl (meth)acrylate, 2-hydroxypentyl (meth)acrylate and 1,4-cyclohexanedimethanol mono(meth)acrylate; polyhydric alcohol (meth)acrylates such as trimethylolpropane mono(meth)acrylate, glycerol mono(meth)acrylate, pentaerythritol di(meth)acrylate, pentaerythritol tri(meth)acrylate, dipentaerythritol di(meth)acrylate, dipentaerythritol tri(meth)acrylate and dipentaerythritol tetra(meth)acrylate;
adducts of aliphatic polyepoxide with (meth) acrylic acid such as adduct of glycidyl (meth)acrylate with (meth)acrylic acid, adduct of dibutylene glycol diglycidyl ether (1 mol) with (meth)acrylic acid (1-2 mol), adduct of polyethylene glycol diglycidyl ether (1 mol) with (meth) acrylic acid (1-2 mol) and adduct of glycerol diglycidyl ether (1 mol) with (meth) acrylic acid (1-2 mol); ε-caprolactam-modified 2-hydroxyethyl (meth)acrylate, ε-caprolactone-modified 2-hydroxyethyl (meth) acrylate, polyethylene glycol mono(meth)acrylate and polypropylene glycol mono (meth)acrylate. These compounds may be used singly, or two or more kinds may be used.

Of the above compounds, those represented by Formula (vii) below are preferable as the compounds (B) having a hydroxyl group and a (meth)acryloyl group. That is, the compound (A) having the skeleton of Formula (3) is preferably a compound obtained by reacting urea, the compound of Formula (vii) and paraformaldehyde.

[Chem. 12]

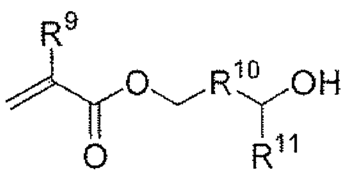

(vii)

In Formula (vii), $R^9$ is a hydrogen atom or a methyl group, $R^{10}$ is a single bond or a C1-6 saturated hydrocarbon group, and $R^{11}$ is a hydrogen atom or a methyl group. In detail, 2-hydroxyethyl (meth)acrylate, 2-hydroxypropyl (meth) acrylate, 3-hydroxybutyl (meth)acrylate, 4-hydroxybutyl (meth)acrylate and 1,4-cyclohexanedimethanol mono(meth) acrylate are preferable because of availability.

Specific examples of the at least one (meth)acryloyl group-containing isocyanate selected from Formulae (iv) to (vi) include 2-acryloyloxyethyl isocyanate, 2-methacryloyloxyethyl isocyanate, 2-(2-methacryloyloxyethyloxy)ethyl isocyanate and 1,1-(bisacryloyloxymethyl)ethyl isocyanate. These isocyanates may be used singly, or two or more kinds may be used.

As described above, the compounds of Formula (vii) having a hydroxyl group and a (meth)acryloyl group include hydroxyalkyl (meth)acrylates such as 2-hydroxyethyl (meth)acrylate, 2-hydroxypropyl (meth)acrylate, 3-hydroxybutyl (meth)acrylate, 4-hydroxybutyl (meth)acrylate and 1,4-cyclohexanedimethanol mono(meth)acrylate. In particular, 2-hydroxyethyl (meth)acrylate, 2-hydroxypropyl (meth)acrylate, 4-hydroxybutyl (meth)acrylate and 1,4-cyclohexanedimethanol mono(meth)acrylate are preferable from the economic point of view. These compounds may be used singly, or two or more kinds may be used.

The at least one (meth)acryloyl group-containing isocyanate selected from Formulae (iv) to (vi) and water may be reacted (reaction I) in any ratio without limitation. Generally, water may be used in a 0.4 to 0. 6-fold equivalent, and preferably a 0.45 to 0.55-fold equivalent based on 1 equivalent of the isocyanate groups in the (meth)acryloyl group-containing isocyanate selected from Formulae (iv) to (vi). If the ratio is outside this range, either of the compounds that is in excess remains unreacted and the step for removing the excess compound is required possibly to cause economic disadvantages. Further, the compounds may be added to the reactor in any order without limitation. In a preferred embodiment, the reaction is performed by adding water dropwise to the at least one (meth)acryloyl group-containing isocyanate selected from Formulae (iv) to (vi). This reaction (the reaction I) is considered to proceed in a manner such that a part of the isocyanate groups react with water to form an amine compound (the first stage) which undergoes a reaction with the remaining isocyanate groups (the second stage).

The at least one (meth)acryloyl group-containing isocyanate selected from Formulae (iv) to (vi) and urea may be reacted (reaction II) in any ratio without limitation. Generally, the at least one (meth)acryloyl group-containing isocyanate selected from Formulae (iv) to (vi) may be used in 1.6 to 2.4 equivalents, and preferably 1.8 to 2.2 equivalents relative to 1 equivalent of the urea. If the ratio is outside this range, either of the compounds that is in excess remains unreacted and economic disadvantages may be possibly caused. Further, the compounds may be added to the reactor in any order without limitation. In a preferred embodiment, the reaction is performed by adding dropwise to the urea the at least one (meth)acryloyl group-containing isocyanate selected from Formulae (iv) to (vi).

Urea, the compound of Formula (vii) having a hydroxyl group and a (meth)acryloyl group and paraformaldehyde may be reacted (reaction III) in any ratio without limitation. Generally, the paraformaldehyde and the compound of Formula (vii) having a hydroxyl group and a (meth)acryloyl group may be used each in 3.6 to 4.4 equivalents, and

preferably 3.8 to 4.2 equivalents based on 1 equivalent of the urea. If the ratio is outside this range, any of the compounds that is in excess remains unreacted and economic disadvantages may be possibly caused. Further, the compounds may be added to the reactor in any order without limitation. In a preferred embodiment, the three compounds are mixed together prior to the reaction.

In the reaction I and the reaction II, a catalyst may be used to shorten the reaction time. Examples of the catalysts include basic catalysts (amines such as pyridine, pyrrole, triethylamine, diethylamine, dibutylamine and ammonia, and phosphines such as tributylphosphine and triphenylphosphine) and acidic catalysts (metal alkoxides such as copper naphthenate, cobalt naphthenate, zinc naphthenate, tributoxyaluminum, tetrabutoxy-trititanium and tetrabutoxyzirconium, Lewis acids such as aluminum chloride, and tin compounds such as dibutyl tin dilaurate and dibutyl tin acetate). Of these, the acidic catalysts are preferred, and the tin compounds are most preferable. The catalysts are usually added in 0.0001 to 5 mol per 1 mol of the at least one (meth)acryloyl group-containing isocyanate selected from Formulae (iv) to (vi).

The reaction temperature in the reaction I and the reaction II is preferably in the range of 5 to 100°C, and more preferably 20 to 40°C. The reaction rate is lowered at a reaction temperature below 5°C. Temperatures above 100°C tend to induce thermal polymerization of the product.

In the reaction III, a catalyst may be used to shorten the reaction time. Examples of the catalysts include organic and inorganic acid catalysts which are commonly used to accelerate the dehydration condensation reaction, with specific examples including paratoluenesulfonic acid, paratoluenesulfonic acid monohydrate and hydrochloric acid. Organic and inorganic basic catalysts such as caustic soda, sodium carbonate, ammonia and triethylamine may be used in order to increase the solubility of melamine and to accelerate the dehydration condensation reaction. Of the catalysts, the acid catalysts are preferable and may be usually added in 0.0005 to 0.025 mol per 1 mol of the urea.

In the reaction III, the reaction temperature is preferably in the range of 20 to 200°C, and more preferably 60 to 120°C. Reaction temperatures below 20°C lower the reaction rate. Above 200°C, thermal polymerization tends to take place.

To prevent gelation during the reactions I to III, a polymerization inhibitor is preferably added. Examples of the inhibitors include phenolic compounds such as hydroquinone, p-methoxyphenol, 2-t-butylhydroquinone, 2-t-butyl-4-methoxyphenol, 2,6-di-tert-butyl-p-cresol, 4-t-butylcatechol, 2,5-di-t-butylhydroquinone, 2,5-di-t-amylhydroquinone, 4,4'-thio-bis(6-t-butyl-m-cresol), 2,6-di-t-butyl-4-ethylphenolstearyl-β-(3,5-di-t-butyl-4-hydroxyphenyl) propionate, 2,2'-methylenebis(4-methyl-6-t-butylphenol), 4,4'-butylidenebis(3-methyl-6-t-butylphenol), 1,3,5-trimethyl-2,4,6-tris(3,5-di-t-butyl-4-hydroxybenzyl) benzene, tetrakis-[methylene-3-(3',5'-di-t-butyl-4'-hydroxyphenyl)propionate]methane, tris(3',5'-di-t-butyl-4'-hydroxybenzyl)-s-triazine-2,4,6-(1H, 3H, 5H) trione and tocopherol; sulfur compounds such as phenothiazine, styryl phenothiazine, dilauryl 3,3'-thiodipropionate, dimyristyl 3,3'-thiodipropionate, distearyl 3,3'-thiodipropionate and copper dibutyldithiocarbamate; and phosphorus compounds such as triphenyl phosphite, diphenylisodecyl phosphite and phenyldiisodecyl phosphite. These inhibitors may be used singly, or two or more kinds may be used in combination. The addition amount thereof is preferably 10 to 10000 ppm, and more preferably 500 to 2000 ppm based on the reaction liquid. The polymerization inhibitors may not provide effects when used in amounts less than 10 ppm. The use thereof in excess of 10000 ppm may adversely affect the curability and color.

The reaction is preferably carried out in the presence of oxygen in order similarly to prevent polymerization. The concentration of oxygen that is present is desirably not more than 10 mol% in terms of the concentration in the gas phase in view of the explosion limit. Oxygen provides polymerization inhibitory effects even at concentrations above 10 mol%, but such concentrations easily lead to fire in the presence of an ignition source.

The reaction may involve solvents. The solvents which may be used include aromatic hydrocarbons such as toluene and xylene, halogenated hydrocarbons such as dichloroethane and chlorobenzene, esters such as ethyl acetate, propyl acetate, butyl acetate and propylene glycol monomethyl ether acetate, ethers such as tetrahydrofuran and γ-butyrolactone, amide solvents such as dimethylformamide, N,N-dimethylacetamide and N-methylpyrrolidone, and sulfoxide solvents such as dimethylsulfoxide.

The curable compositions for transfer materials according to the invention contain the compound (A) resulting from the above-mentioned reaction, which has at least one skeleton selected from Formulae (1) to (3) and a (meth) acryloyl group. Because the compound (A) has the radically polymerizable functional group, radical polymerization or radical addition polymerization involving polyaddition of polythiol is possible.

Including the compound (A) which has at least one skeleton selected from Formulae (1) to (3) and a (meth) acryloyl group, the curable compositions for transfer materials of the invention achieve excellent transfer performance in the imprinting, and are easily removed by reactive ion etching with oxygen gas when the imprinted composition is to be perforated or removed and also show resistance in etching steps such as Ar milling in the processing of base-medium substrates. Further, because the curable compositions for transfer materials of the invention contain a (meth)acryloyl group as a functional group, radical polymerization is possible.

Specific examples of the compounds (A) include (meth) acryloyl group-containing urea compounds represented by Formula (6) below.

[Chem. 13]

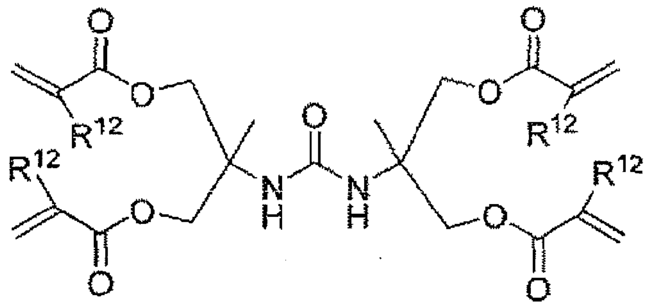

(6)

In Formula (6), $R^{12}$ represents a hydrogen atom or a methyl group.

The (meth)acryloyl group-containing urea compound of Formula (6) may be produced by, for example, the reaction (I) described above. In detail, the compound may be obtained by stirring 1,1-(bisacryloyloxymethyl) ethyl isocyanate and water at room temperature in the presence of dibutyl tin dilaurate and 2,6-di-tert-butyl-p-cresol in an air atmosphere.

(Other components in curable compositions for transfer materials)

The curable compositions for transfer materials of the invention contain the compound (A) and may further contain a radically polymerizable compound other than the compound (A).

In the curable compositions for transfer materials, the curable functional group in the compound (A) is the (meth)acryloyl group. Thus, the compound (A) can undergo radical polymerization. Further, the curable compositions for transfer materials may contain another radically polymerizable compound having a functional group copolymerizable with the (meth) acryloyl group in the compound (A) . The compound (A) may be radically polymerized in the presence of such a compound. In an embodiment, the curable composition for transfer materials may contain a polythiol as the radically polymerizable compound other than the compound (A). In such a case, the compound (A) may undergo radical polymerization in combination with polyaddition in the presence of the polythiol. The use of radically polymerizable compounds other than the compounds (A) enables controlling the curing rate, the viscosity and the like of the curable compositions for transfer materials of the invention.

When the curable composition for transfer materials contains a radically polymerizable compound other than the compound (A), the amount thereof is preferably 0.1 to 50 parts by mass based on 100 parts by mass of the curable composition for transfer materials.

Examples of the compounds having a functional group copolymerizable with the (meth)acryloyl group include compounds having such groups as (meth)acryloyl group, styryl group, vinyl group, allyl group, maleate group and fumarate group. Of these, compounds having a (meth)acryloyl group are particularly preferable, and monomers or oligomers having one or more (meth)acrylate structures are suitably used.

The monomers or oligomers having one or more (meth)acryloylate structures may be monofunctional or polyfunctional (meth)acrylates. Specific examples include methyl (meth)acrylate, ethyl (meth)acrylate, n-propyl (meth)acrylate, isopropyl (meth)acrylate, n-butyl (meth)acrylate, isobutyl (meth)acrylate, sec-butyl (meth)acrylate, hexyl (meth) acrylate, octyl (meth)acrylate, 2-ethylhexyl (meth)acrylate, decyl (meth)acrylate, isobornyl (meth)acrylate, cyclohexyl (meth)acrylate, phenyl (meth)acrylate, benzyl (meth)acrylate, 2-hydroxyethyl (meth)acrylate, 2-hydroxypropyl (meth) acrylate, 3-hydroxypropyl (meth)acrylate, 2-hydroxybutyl (meth)acrylate, 2-hydroxyphenylethyl (meth)acrylate, ethylene glycol di(meth)acrylate, propylene glycol di(meth)acrylate, 1,4-butanediol di(meth)acrylate, diethylene glycol di(meth) acrylate, triethylene glycol di(meth)acrylate, trimethylolpropane di(meth)acrylate, trimethylolpropane tri(meth)acrylate and pentaerythritol penta(meth)acrylate. Further, monomers having one (meth) acrylamide structure with a (meth) acryloyl group may be used as the compounds having a functional group copolymerizable with the (meth) acryloyl group similarly to the (meth) acrylates. Specific examples of such compounds include N,N-dimethyl (meth)acrylamide, N,N-diethyl (meth)acrylamide and N-acryloylmorpholine.

Examples of the compounds having a functional group copolymerizable with the (meth) acryloyl group further include epoxy acrylates that are adducts of epoxy resins with (meth)acrylic acid, such as bisphenol A epoxy resins, hydrogenated bisphenol A epoxy resins, brominated bisphenol A epoxy resins, bisphenol F epoxy resins, novolac epoxy resins, phenol novolac epoxy resins, cresol novolac epoxy resins, alicyclic epoxy resins, N-glycidyl epoxy resins, bisphenol A novolac epoxy resins, chelated epoxy resins, glyoxal epoxy resins, amino group-containing epoxy resins, rubber-modified epoxy resins, dicyclopentadiene phenolic epoxy resins, silicone-modified epoxy resins and ε-caprolactone-modified epoxy resins.

Examples of the compounds having a functional group copolymerizable with the (meth) acryloyl group further include urethane acrylates resulting from the reaction of a polyisocyanate compound with an active hydrogen-containing (meth)acrylate monomer.

Examples of the polyisocyanate compounds include 2, 4- or 2,6-tolylene diisocyanate, m- or p-xylylene diisocyanate, hydrogenated xylylene diisocyanate, diphenylmethane-4,4'-diisocyanate and modified products thereof and polymers thereof, hexamethylene diisocyanate, isophorone diisocyanate, 1,4-tetramethylene diisocyanate and naphthalene diisocyanate.

Examples of the active hydrogen-containing (meth) acrylate monomers include 2-hydroxyethyl (meth) acrylate, 2-hydroxypropyl (meth)acrylate, tripropylene glycol (meth)acrylate, 1,4-butylene glycol mono(meth)acrylate, 2-hydroxy-3-chloropropyl (meth)acrylate, glycerol mono(meth)acrylate, glycerol di(meth)acrylate, glycerol methacrylate acrylate, trimethylolpropane di(meth)acrylate and pentaerythritol tri(meth)acrylate.

Styrene and derivatives thereof are also employable as the compounds having a functional group copolymerizable with the (meth)acryloyl group. Examples include styrene, 2,4-dimethyl-α-methylstyrene, o-methylstyrene, m-methylstyrene, p-methylstyrene, 2,4-dimethylstyrene, 2,5-dimethylstyrene, 2,6-dimethylstyrene, 3,4-dimethylstyrene, 3,5-dimethylstyrene, 2,4,6-trimethylstyrene, 2,4,5-trimethylstyrene, pentamethylstyrene, o-ethylstyrene, m-ethylstyrene, p-ethylstyrene, o-chlorostyrene, m-chlorostyrene, p-chlorostyrene, o-bromostyrene, m-bromostyrene, p-bromostyrene, o-methoxystyrene, m-methoxystyrene, p-methoxystyrene, o-hydroxystyrene, m-hydroxystyrene, p-hydroxystyrene, 2-vinylbiphenyl, 3-vinylbiphenyl, 4-vinylbiphenyl, 1-vinylnaphthalene, 2-vinylnaphthalene, 4-vinyl-p-terphenyl, 1-vinyl anthracene, α-methylstyrene, o-isopropenyltoluene, m-isopropenyltoluene, p-isopropenyltoluene, 2,4-dimethyl-α-methylstyrene, 2,3-dimethyl-α-methylstyrene, 3,5-dimethyl-α-methylstyrene, p-isopropyl-α-methylstyrene, α-ethylstyrene, α-chlorostyrene, divinylbenzene, divinylbiphenyl and diisopropylbenzene.

Further, (meth) acrylonitrile and derivatives thereof are also employable as the compounds having a functional group copolymerizable with the (meth)acryloyl group. Examples include acrylonitrile and methacrylonitrile.

Examples of the compounds having a functional group copolymerizable with the (meth) acryloyl group further include vinyl esters of organic carboxylic acids and derivatives thereof such as vinyl acetate, vinyl propionate, vinyl butyrate, vinyl benzoate and divinyl adipate.

Examples of the compounds having a functional group copolymerizable with the (meth)acryloyl group furthermore include allyl esters of organic carboxylic acids and derivatives thereof such as allyl acetate, allyl benzoate, diallyl adipate, diallyl terephthalate, diallyl isophthalate and diallyl phthalate.

Dialkyl esters of fumaric acid and derivatives thereof are also employable as the compounds having a functional group copolymerizable with the (meth)acryloyl group. Examples include dimethyl fumarate, diethyl fumarate, diisopropyl fumarate, di-sec-butyl fumarate, diisobutyl fumarate, di-n-butyl fumarate, di-2-ethylhexyl fumarate and dibenzyl fumarate.

Dialkyl esters of maleic acid and derivatives thereof are also employable as the compounds having a functional group copolymerizable with the (meth)acryloyl group. Examples include dimethyl maleate, diethyl maleate, diisopropyl maleate, di-sec-butyl maleate, diisobutyl maleate, di-n-butyl maleate, di-2-ethylhexyl maleate and dibenzyl maleate.

Examples of the compounds having a functional group copolymerizable with the (meth) acryloyl group further include dialkyl esters of itaconic acid and derivatives thereof such as dimethyl itaconate, diethyl itaconate, diisopropyl itaconate, di-sec-butyl itaconate, diisobutyl itaconate, di-n-butyl itaconate, di-2-ethylhexyl itaconate and dibenzyl itaconate.

Examples of the compounds having a functional group copolymerizable with the (meth)acryloyl group furthermore include N-vinylamide derivatives of organic carboxylic acids such as N-methyl-N-vinylacetamide.

Further, maleimide and derivatives thereof such as N-phenylmaleimide and N-cyclohexylmaleimide are employable as the compounds having a functional group copolymerizable with the (meth)acryloyl group.

In the radical polymerization in combination with the polyaddition of the compound (A) and a polythiol, examples of the polythiols which may be used in combination with the compound (A) include 2,2-bis(mercaptomethyl)-1,3-propanedithiol, bis(2-mercaptoethyl)ether, ethylene glycol bis(2-mercaptoacetate), ethylene glycol bis(3-mercaptopropionate), trimethylolpropane tris-(β-thiopropionate), tris-2-hydroxyethyl isocyanurate·tris-β-mercaptopropionate, pentaerythritol tetrakis(β-thiopropionate), 1,8-dimercapto-3,6-dioxaoctane, 1,2,3-trimercaptobenzene, 1,2,4-trimercaptobenzene, 1,3,5-trimercaptobenzene, 1,2,3-tris(mercaptomethyl)benzene, 1,2,4-tris(mercaptomethyl)benzene and 1,3,5-tris(mercaptomethyl)benzene.

When the curable composition for transfer materials is cured by the radical polymerization, two possible curing methods are active energy ray curing and thermal curing. The curable composition for transfer materials desirably contains an appropriate polymerization initiator depending on the polymerization mode.

In the case where the curable functional group is the (meth) acryloyl group and the curable composition for transfer materials contains the polythiol compound, it is preferable that an active energy ray radical polymerization initiator is used as the polymerization initiator.

When the curable composition for transfer materials is cured by the thermal curing, namely in the case of thermal polymerization, a thermal radical polymerization initiator may be used as the polymerization initiator. Examples of the thermal radical polymerization initiators include organic peroxides such as methyl ethyl ketone peroxide, cyclohexanone peroxide, methylcyclohexanone peroxide, methyl acetate peroxide, acetyl acetate peroxide, 1,1-bis(t-butylperoxy) butane, 1,1-bis(t-butylperoxy)-cyclohexane, 1,1-bis(t-butylperoxy)-2-methylcyclohexane, 1,1-bis(t-butylperoxy)-3,3,5-trimethylcyclohexane, 1,1-bis(t-butylperoxy)cyclododecane, 1,1-bis(t-hexylperoxy)-cyclohexane, 1,1-bis(t-hexylperoxy)-3,3,5-trimethylcyclohexane, 2,2-bis(4,4-di-t-butylperoxycyclohexyl)propane, t-butyl hydroperoxide, t-hexyl hydroperoxide, 1,1,3,3-tetramethylbutyl hydroperoxide, cumene hydroperoxide, p-methyl hydroperoxide, diisopropylbenzene hydroperoxide, di-t-butyl peroxide, dicumyl peroxide, t-butylcumyl peroxide, α,α'-bis(t-butylperoxy)diisopropylbenzene, 2,5-dimethyl-2,5-bis(t-butylperoxy)hexane, 2,5-dimethyl-2,5-bis(t-butylperoxy)hexyne-3, isobutyryl peroxide, 3, 3, 5-trimethylhexanoyl peroxide, octanoyl peroxide, lauroyl peroxide, stearoyl peroxide, succinic acid peroxide, m-toluoyl benzoyl peroxide, benzoyl peroxide, di-n-propyl peroxydicarbonate, diisopropyl peroxydicarbonate, bis(4-t-butylcyclohexyl) peroxydicarbonate, di-2-ethoxyethyl peroxydicarbonate, di-2-ethoxyhexyl peroxydicarbonate, di-3-methoxybutyl peroxydicarbonate, di-s-butyl peroxydicarbonate, di(3-methyl-3-methoxybutyl) peroxydicarbonate, α,α'-bis(neodecanoylperoxy)diisopropylbenzene, t-butyl peroxyneodecanoate, t-hexyl peroxyneodecanoate, 1,1,3,3-tetramethylbutyl peroxyneodecanoate, 1-cyclohexyl-1-methylethyl peroxyneodecanoate, cumyl peroxyneodecanoate, t-butyl peroxypivalate, t-hexyl peroxypivalate, t-butyl peroxy-2-ethylhexanoate, t-hexyl peroxy-2-ethylhexanoate, 1,1,3,3-tetramethylbutyl peroxy-2-ethylhexanoate, 2,5-dimethyl-2,5-bis(2-ethylhexanoylperoxy)hexane, 1-cyclohexyl-1-methylethyl peroxy-2-ethylhexanoate, t-butyl peroxy-3,5,5-trimethylhexanoate, t-butylperoxyisopropyl monocarbonate, t-hexylperoxyisopropyl monocarbonate, t-butylperoxy-2-ethylhexyl monocarbonate, t-butylperoxyallyl monocarbonate, t-butyl peroxyisobutyrate, t-butyl peroxymaleate, t-butyl peroxybenzoate, t-hexyl peroxybenzoate, t-butyl peroxy-m-toluylbenzoate, t-butyl peroxylaurate, t-butyl peroxyacetate, bis(t-butylperoxy)isophthalate, 2,5-dimethyl-2,5-bis(m-toluylperoxy)hexane, 2,5-dimethyl-2,5-bis(benzoylperoxy)hexane, t-butyltrimethylsilyl peroxide and 3,3',4,4'-tetra(t-butylperoxycarbonyl)benzophenone; and

azo compounds such as 1-[(1-cyano-1-methylethyl)azo]formamide, 1,1'-azobis(cyclohexane-1-carbonitrile), 2,2'-azobis (2-methylbutyronitrile), 2,2'-azobisisobutyronitrile, 2,2'-azobis(2,4-dimethyl-4-methoxyvaleronitrile), 2,2'-azobis(2,4-dimethylvaleronitrile), 2-phenylazo-4-methoxy-2,4-dimethylvaleronitrile, 2,2'-azobis(2-methylpropionamidine)dihydrochloride, 2,2-azobis(2-methyl-N-phenylpropionamidine)dihydrochloride, 2,2'-azobis[N-(4-chlorophenyl)-2-methylpropionamidine] dihydrochloride, 2,2'-azobis[N-(4-hydrophenyl)-2-methylpropionamidine] dihydrochloride, 2,2'-azobis[2-methyl-N-(2-propenyl)propionamidine] dihydrochloride, 2,2'-azobis[N-(2-hydroxyethyl)-2-methylpropionamidine] dihydrochloride, 2,2'-azobis[2-methyl-N-(phenylmethyl)propionamidine] dihydrochloride, 2,2'-azobis[2-(2-imidazolin-2-yl)propane] dihydrochloride, 2,2'-azobis[2-(5-methyl-2-imidazolin-2-yl)propane] dihydrochloride, 2,2'-azobis{2-[1-(2-hydroxyethyl)-2-imidazolin-2-yl] propane} dihydrochloride, 2,2'-azobis[2-(4,5,6,7-tetrahydro-1H-1,3-diazepin-2-yl) propane] dihydrochloride, 2,2'-azobis[2-(3,4,5,6-tetrahydropyrimidin-2-yl)propane] dihydrochloride, 2,2'-azobis[2-(5-hydroxy-3,4,5,6-tetrahydropyrimidin-2-yl) propane] dihydrochloride, 2,2'-azobis(2-methylpropionamide), 2,2'-azobis[2-methyl-N-(2-hydroxyethyl)propionamide], 2,2'-azobis{2-methyl-N-[1,1-bis(hydroxymethyl)-2-hydroxyethyl]propionamide}, 2,2'-azobis{2-methyl-N-[1,1-bis(hydroxymethyl)ethyl] propionamide}, 2,2'-azobis(2-methylpropane), 2,2'-azobis(2,4,4-trimethylpentane), dimethyl-2,2'-azobis(2-methylpropionate), 4,4'-azobis(4-cyanopentanoic acid) and 2,2'-azobis[2-(hydroxymethyl)propionitrile].

The active energy rays used for the active energy ray curing of the curable compositions are not particularly limited as long as the rays can cure the curable composition for transfer materials by acting on the functional groups of the compound (A) having at least one skeleton selected from Formulae (1) to (3) and a (meth)acryloyl group. Examples of the active energy rays include radiations such as UV rays and X-rays, and electron beams. Of these, UV rays and electron beams may be suitably used.

The electron beams can induce a reaction of the (meth) acryloyl groups even in the absence of the polymerization initiators, thereby the composition being cured. The curable compositions for transfer materials desirably contain an active energy ray radical polymerization initiator as required depending on the combination of the active energy ray applied to the composition and the functional group in the composition.

Examples of the active energy ray radical polymerization initiators desirably used in the curable compositions for transfer materials of the invention include acetophenone radical photopolymerization initiators such as 4-phenoxydichloroacetophenone, 4-t-butyl-dichloroacetophenone, 4-t-butyl-trichloroacetophenone, diethoxyacetophenone, 2-hydroxy-2-cyclohexylacetophenone, 2-hydroxy-2-methyl-1-phenylpropan-1-one, 2-hydroxy-2-phenyl-1-phenylpropan-1-one, 1-(4-dodecylphenyl)-2-hydroxy-2-methylpropan-1-one, 1-(4-isopropylphenyl)-2-hydroxy-2-methylpropan-1-one, 4-(2-hydroxyethoxy)-phenyl-(2-hydroxy-2-propyl) ketone, 1-hydroxycyclohexyl phenyl ketone and 2-methyl-1-[4-(methylthio)phenyl]-2-morpholinopropan-1;
benzoin radical photopolymerization initiators such as benzoin, benzoin methyl ether, benzoin isopropyl ether, benzoin isobutyl ether and benzyl methyl ketal;
benzophenone radical photopolymerization initiators such as benzophenone, benzoylbenzoic acid, methyl benzoylbenzoate, 4-phenylbenzophenone, hydroxybenzophenone, acrylated benzophenone, 4-benzoyl-4'-methyldiphenyl sulfide, 3,3'-dimethyl-4-methoxybenzophenone, 4,4'-dimethylaminobenzophenone, 4,4'-diethylaminobenzophenone and 3,3', 4,4'-tetra(t-butylperoxycarbonyl)benzophenone;
thioxanthone radical photopolymerization initiators such as thioxanthone, 2-chlorothioxanthone, 2-methylthioxanthone, 2,4-dimethylthioxanthone, 2,4-diethylthioxanthone, 2,4-diisopropylthioxanthone, isopropylthioxanthone, 1-chloro-4-propoxythioxanthone and 2,4-dichlorothioxanthone;
ketone radical photopolymerization initiators such as α-acyloxime esters, methyl phenyl glyoxylate, benzil, 9,10-phenanthrenequinone, camphorquinone, dibenzosuberone, 2-ethylanthraquinone and 4',4" -diethylisophthalophenone;
imidazol radical photopolymerization initiators such as 2,2'-bis(2-chlorophenyl)-4,4',5,5'-tetraphenyl-1,2'-imidazol;
acylphosphine oxide radical photopolymerization initiators such as 2,4,6-trimethylbenzoyldiphenylphosphine oxide;
carbazole radical photopolymerization initiators;
Lewis acid onium salt radical photopolymerization initiators such astriphenylphosphonium hexafluoroantimonate, triphenylphosphonium hexafluorophosphate, p-(phenylthio)phenyldiphenylsulfonium hexafluoroantimonate, 4-chlorophenyldiphenylsulfonium hexafluorophosphate and (2,4-cyclopentadien-1-yl)[(1-methylethyl)benzene]-iron-hexafluorophosphate; and
α-aminoalkylphenone radical photopolymerization initiators such as 2-benzyl-2-dimethylamino-1-(4-morpholinophenyl)-1-butanone and 2-methyl-1-[4-(methylthio)phenyl]-2-morpholinopropan-1-one.

These polymerization initiators may be used singly, or two or more kinds may be used in combination. The amount thereof is preferably 0.01 to 10 parts by mass based on 100 parts by mass of the curable composition for transfer materials.

The curable compositions for transfer materials according to the invention may further contain additives such as viscosity modifiers, dispersants and surface conditioners. When such additives are added, the total amount of the additives is preferably not more than 30 parts by mass based on 100 parts by mass of the whole curable composition. If the amount of the additives is excessively large, fine patterns obtainable from the curable composition may have deteriorated etching properties.

The curable compositions for transfer materials may contain solvents or the like as required in order to improve application properties in the case of formation of fine patterns. Herein, the solvent used in the reaction for the production of the compound (A) may be continuously used. Alternatively, the reaction solvent may be distilled away under reduced pressure and the residue may be diluted with a different solvent. Examples of the solvents include ketone solvents such as methyl isobutyl ketone, aromatic hydrocarbon solvents such as toluene and xylene, ester solvents such as ethyl acetate, butyl acetate and propylene glycol monomethyl ether acetate, alcohol solvents such as 2-propanol, butanol, hexanol propylene glycol mono-n-propyl ether and ethylene glycol monoethyl ether, and amide solvents such as N,N-dimethylacetamide, dimethylformamide and n-methylpyrrolidone.

[Pattern forming processes]

Next, there will be described processes for forming a pattern using the curable composition for transfer materials according to the invention. In particular, fine patterns of 10 μm or less may be fabricated according to the processes. Herein, the fine patterns of 10 μm or less are patterns which are imprinted with a mold having projections and depressions with a linewidth of 10 μm or less. That is, one depression and one projection have a total linewidth (pitch) of not more than 10 μm. The lower limit of the pitches in the patterns produced by the inventive pattern forming processes is not particularly limited. However, the pitches are usually 30 nm or more.

In one embodiment, the pattern forming process according to the invention includes a step of applying the curable composition for transfer materials to a substrate to form a coating layer (also referred to as the application step); a step of pressing a mold having a surface with a pattern of projections and depressions into the coating layer (also referred to as the transfer step) ; a step of heating the coating layer while keeping the mold pressed in the coating layer, thereby to cure the coating layer (also referred to as the curing step) ; and a step of separating the mold from the cured coating layer (also referred to as the separation step).

In another embodiment, the pattern forming process includes a step of applying the curable composition for transfer materials to a substrate to form a coating layer (also referred to as the application step); a step of pressing a mold having a surface with a pattern of projections and depressions into the coating layer (also referred to as the transfer step); a step of applying an active energy ray to the coating layer while keeping the mold pressed in the coating layer,

thereby to cure the coating layer (also referred to as the curing step); and a step of separating the mold from the cured coating layer (also referred to as the separation step).

[1. Application step]

The curable composition for transfer materials may be applied to a substrate by any methods without limitation, for example by spin coating or dip coating. It is preferable to adopt a method capable of spreading the curable composition on a substrate to form a coating layer in a uniform thickness. Fig. 1 (a) illustrates the curable composition for transfer materials of the invention that has been applied on a substrate, in detail a coating layer 14 formed on a substrate 16. The substrate herein refers to a single base such as a glass plate or a multilayer in which a layer to be patterned such as a magnetic layer and/or a protective layer is provided on the base.

[2. Transfer step and curing step]

A pattern may be fabricated by pressing a patterned mold into the coating layer (thereby transferring the pattern). In the invention, a mold is used which has a surface with a pattern of projections and depressions, and the surface of the mold is pressed against the coating layer. After the mold is pressed into the coating layer, the curable composition is cured with active energy rays or by heating, or both in combination, namely by applying active energy rays under heating. Figs. 1 (b) and (c) show steps in which a mold is pressed into the curable composition that has been applied to a substrate, and the composition is cured by irradiation of active energy rays and/or by heating. Fig. 1 (b) illustrates that a mold 12 is in pressed contact with the coating layer 14 on the substrate 16. In Fig. 1 (c), active energy rays are applied to the coating layer 14 and/or heat 18 is applied thereto while the mold 12 is kept in pressed contact with the coating layer 14.

The mold may be formed of any materials without limitation. However, when the curable composition for transfer materials is cured with active energy rays such as UV rays, it is necessary that at least the mold is made of a material capable of transmitting active energy rays or at least the substrate is composed of a material which transmits active energy rays.

When use is made of a mold composed of a material capable of transmitting active energy rays, it is a preferred embodiment that the mold is made of a resin, glass or quartz which transmits active energy rays because the active energy rays can be transmitted to the coating layer through the mold from the side of the mold that is opposite the side thereof in contact with the coating layer and thereby the curable composition can be cured and form a pattern even when the substrate used does not transmit active energy rays. That is, referring to Fig. 1 (c), active energy rays are applied from above the mold 12 and reach the finely patterned coating layer 14 through the mold 12, whereby the curable composition is cured to form a pattern.

In another preferred embodiment, use is made of a transparent substrate that transmits active energy rays. In such embodiments, active energy rays are usually applied to the coating layer through the substrate from the side of the substrate that is opposite the side thereof in contact with the coating layer, and thereby the curable composition is cured to form a pattern. That is, referring to Fig. 1 (c), active energy rays are applied from below the substrate 16 and reach the finely patterned coating layer 14 through the substrate 16, whereby the curable composition is cured to form a pattern.

As described above, the active energy rays used in the invention are not particularly limited as long as they can induce the curing of the composition by acting on the curable functional groups in the compound (A). Suitable active energy rays include UV rays and electron beams.

Any atmosphere may be used without limitation in pressing the mold into the composition or in the subsequent heating or application of the energy rays thereto. However, a vacuum atmosphere is preferable to prevent any bubbles from remaining in the cured product from the curable composition. When the curable functional groups are carbon-carbon double bonds such as in (meth)acryloyl groups, allyl groups or vinyl groups, the mold pressing and the subsequent heating or energy ray irradiation are preferably performed in vacuum to prevent polymerization inhibition by oxygen.

[3. Separation step]

After the coating layer of the curable composition is cured, the mold is separated from the coating layer. Fig. 1 (d) illustrates the cured and patterned coating layer 14, the mold 12 having been separated therefrom. After the mold is separated, the layer may be heated to increase the heat resistance or the physical strength of the pattern. Here, the heating method is not particularly limited. In a preferred embodiment, the temperature is slowly increased but is kept below the glass transition temperature of the coating layer to avoid the deformation of the pattern, and the upper limit of the heating temperature is 250°C to prevent thermal decomposition of the coating layer.

Patterns may be fabricated as described above. The pattern forming processes of the invention can produce

fine patterns of 10 μm or less. The fine patterns are the cured products of the curable composition for transfer materials according to the invention. The fine patterns have very high transfer fidelity and show high selectivity of etching rate between by argon gas and by oxygen gas. In detail, the fine patterns of 10 μm or less that are fabricated by the fine pattern forming process of the invention have high resistance against etching gas (argon gas) to facilitate controlling the etching degree, and meanwhile have low resistance against gas used for the removal thereof (oxygen gas) to permit easy removal. Accordingly, the fine patterns can make excellent resists, finding wide use in applications such as semiconductors and magnetic recording media.

As described above, the curable compositions for transfer materials of the invention may be used in wide applications including magnetic recording media. In detail, the fine patterns may be used in the processing for partial removal or partial demagnetization of a magnetic layer of a magnetic recording medium. Specifically, a process for manufacturing magnetic recording media according to the invention is **characterized in that** a substrate including a base and a magnetic layer thereon is provided and a pattern is formed on the magnetic layer by the above-described pattern forming process; and the magnetic layer is partially removed or partially demagnetized using the pattern as a resist. A magnetic recording medium according to the invention is obtained by the process for manufacturing magnetic recording media.

An embodiment of the process for manufacturing magnetic recording media according to the invention will be described below.

(1. Perforation)

In the process for manufacturing magnetic recording media, a substrate including a base and a magnetic layer thereon is provided and a pattern is formed on the magnetic layer by the above-described pattern forming process. Thereafter, the pattern is subjected to ion etching (RIE, or ion milling) to etch the depressions in the projection/depression pattern, thereby exposing the surface of the magnetic layer. Fig. 3 schematically illustrates this step. Referring to Fig. 3, a substrate includes a base 22 and a magnetic layer 24 thereon, and a cured layer 26 which has a fine pattern formed from the curable composition of the invention is provided on the magnetic layer. Reactive ion etching 20 is performed from above the cured layer (the upper illustration in Fig. 3). As a result, the depressions in the fine pattern of the cured layer are etched and consequently the cured layer on the magnetic layer is removed corresponding to the fine pattern (the lower illustration in Fig. 3).

(2. Partial removal or partial demagnetization of magnetic layer)

The exposed magnetic layer is removed by ion milling or is demagnetized with a reactive gas, using the pattern as a resist.

Here, the pattern should be resistant to ion milling and reactive gas. Fig. 4 schematically illustrates this step. The left illustration in Fig. 4 shows an ion milling (ion etching) step, in which ion milling 28 is performed from above the finely patterned and perforated cured layer (the upper left illustration in Fig. 4) and thereby the magnetic layer exposed from the cured layer is etched (the lower left illustration in Fig. 4). The right illustration in Fig. 4 shows a demagnetization step using a reactive gas, in which the finely patterned and perforated cured layer is processed with a reactive gas 30 from thereabove (the upper right illustration in Fig. 4) and thereby the magnetic layer exposed from the cured layer is demagnetized to a nonmagnetic layer 32 (the lower right illustration in Fig. 4). For example, the magnetic parts may be demagnetized by a method as described in JP-A-2007-273067 in which atoms such as silicon, boron, fluorine, phosphorus, tungsten, carbon, indium, germanium, bismuth, krypton and argon are injected to the magnetic parts by an ion beam method or the like to render the magnetic parts amorphous.

The layer of the cured composition is thereafter removed, and a magnetic recording medium is thus manufactured.

The incorporation of the magnetic recording media produced by the above process into magnetic recording and reading apparatuses provides a drastically increased recording density of the magnetic recording and reading apparatuses while ensuring recording/reading properties that are comparable or superior to the conventional level.

EXAMPLES

The present invention will be described in detail by presenting examples hereinbelow without limiting the scope of the invention.

[Example 1]

A 300 ml three-necked flask equipped with a thermometer and a stirrer was charged with 5.0 g (35.4 mmol) of

2-acryloyloxyethyl isocyanate (KARENZ AOI manufactured by SHOWA DENKO K.K.), 10.0 mg (1.58 x $10^{-2}$ mmol) of dibutyl tin dilaurate, 5 mg (1.88 x $10^{-5}$ mmol) of 2, 6-di-tert-butyl-p-cresol and 0.319 g (17.7 mmol) of water. The materials were stirred at room temperature in an air atmosphere. A white solid was gradually precipitated. After 2 hours, the reaction completed and the precipitation of the solid ceased. White solid powder (a) (acryloyl group-containing urea compound (a)) was thus obtained. The chemical formula of the acryloyl group-containing urea compound (a) is shown in Formula (a) below.

[Chem. 14]

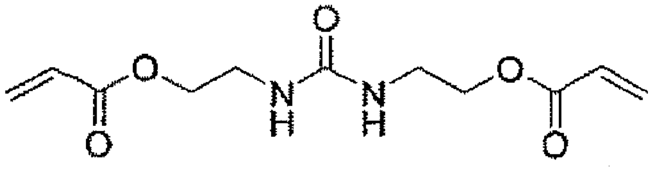

(a)

950 parts by mass of N, N-dimethylacetamide and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white solid powder (a) to dissolve the same. The resultant solution was filtered through a 0.2 μm filter to give a curable composition for transfer materials. The curable composition in a volume of 0.5 ml was dropped onto a glass substrate set in a spin coater, and the glass substrate was rotated at 500 rpm for 5 seconds, 3000 rpm for 2 seconds and 5000 rpm for 20 seconds, thereby forming a thin layer (in a thickness of approximately 0.2 μm) on the glass substrate. UV ray (365 nm wavelength and 35 mW/$cm^2$ dose) was applied to the thin layer of the curable composition formed on the glass substrate, from the thin layer side for 15 seconds in a stream of nitrogen. The resultant thin resin layer was etched with argon gas and oxygen gas to determine the reactive ion etching rates by these gases.

[Example 2]

A 300 ml three-necked flask equipped with a thermometer and a stirrer was charged with 5.0 g (32.2 mmol) of methacryloyloxyethyl isocyanate (KARENZ MOI manufactured by SHOWA DENKO K.K.), 10.0 mg (1.58 x $10^{-2}$ mmol) of dibutyl tin dilaurate, 5 mg (1. 88 x $10^{-5}$ mmol) of 2, 6-di-tert-butyl-p-cresol and 0.290 g (16.1 mmol) of water. The materials were stirred at room temperature in an air atmosphere. A white solid was gradually precipitated. After 2 hours, the reaction completed and the precipitation of the solid ceased. White solid powder (b) (methacryloyl group-containing urea compound (b)) was thus obtained. The chemical formula of the acryloyl group-containing urea compound (b) is shown in Formula (b) below.

[Chem. 15]

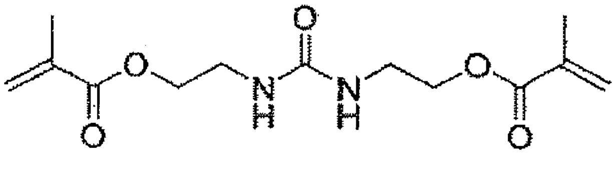

(b)

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white solid powder (b) to dissolve the same. The resultant solution was filtered through a 0.2 μm filter to give a curable composition for transfer materials. The curable composition in a volume of 0.5 ml was dropped onto a glass substrate set in a spin coater, and the glass substrate was rotated at 500 rpm for 5 seconds, 3000 rpm for

2 seconds and 5000 rpm for 20 seconds, thereby forming a thin layer (in a thickness of approximately 0.2 μm) on the glass substrate. UV ray (365 nm wavelength and 35 mW/cm$^2$ dose) was applied to the thin layer of the curable composition formed on the glass substrate, from the thin layer side for 15 seconds in a stream of nitrogen. The resultant thin resin layer was etched with argon gas and oxygen gas to determine the reactive ion etching rates by these gases.

[Example 3]

A 300 ml three-necked flask equipped with a thermometer and a stirrer was charged with 5.0 g (20.9 mmol) of 1,1-(bisacryloyloxymethyl)ethyl isocyanate (KARENZ BEI manufactured by SHOWA DENKO K.K.), 10.0 mg (1.58 x 10$^{-2}$ mmol) of dibutyl tin dilaurate, 5 mg (1.88 x 10$^{-5}$ mmol) of 2,6-di-tert-butyl-p-cresol and 0.188 g (10.5 mmol) of water. The materials were stirred at room temperature in an air atmosphere. A white solid was gradually precipitated. After 2 hours, the reaction completed and the precipitation of the solid ceased. White solid powder (c) (acryloyl group-containing urea compound (c)) was thus obtained.

A $^1$H-NMR spectrum and a $^{13}$C-NMR spectrum of the white solid powder (c) are shown in Figs. 5 and 6, respectively. Based on the results, the white solid powder was identified to be a structure represented by Formula (c) below. (In the $^1$H-NMR spectrum, the peak at 1.42 ppm is assigned to the hydrogen atoms (6H) in the methyl groups in the compound, the peaks at 4.08-4.42 ppm are assigned to the hydrogen atoms (8H) in the methylene groups adjacent to the oxygen atoms, the peak at 4.79 ppm is assigned to the hydrogen atoms (2H) on the nitrogen atoms, and the peaks at 5.85-6.45 ppm are assigned to the hydrogen atoms (12H) on the carbon-carbon double bonds. The $^{13}$C-NMR spectrum indicated seven peaks in agreement with the number of peaks of the expected product.)

[Chem. 16]

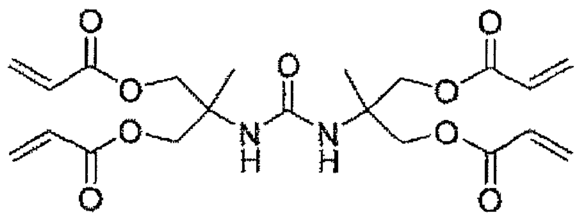

(c)

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white solid powder (c) to dissolve the same. The resultant solution was filtered through a 0.2 μm filter to give a curable composition for transfer materials. The curable composition in a volume of 0.5 ml was dropped onto a glass substrate set in a spin coater, and the glass substrate was rotated at 500 rpm for 5 seconds, 3000 rpm for 2 seconds and 5000 rpm for 20 seconds, thereby forming a thin layer (in a thickness of approximately 0.2 μm) on the glass substrate. UV ray (365 nm wavelength and 35 mW/cm$^2$ dose) was applied to the thin layer of the curable composition formed on the glass substrate, from the thin layer side for 15 seconds in a stream of nitrogen. The resultant thin resin layer was etched with argon gas and oxygen gas to determine the reactive ion etching rates by these gases.

[Comparative Example 1]

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of dipentaerythritol hexaacrylate (KAYARAD DPHA manufactured by NIPPON KAYAKU Co., Ltd.) to dissolve the same. The solution was filtered through a 0.2 μm filter. The resultant curable composition for transfer materials in a volume of 0.5 ml was dropped onto a glass substrate set in a spin coater, and the glass substrate was rotated at 500 rpm for 5 seconds, 3000 rpm for 2 seconds and 5000 rpm for 20 seconds, thereby forming a thin layer (in a thickness of approximately 0.2 μm) on the glass substrate. UV ray (365 nm wavelength and 35 mW/cm$^2$ dose) was applied to the resin on the glass substrate, from the thin layer side for 15 seconds in a stream of nitrogen. The resultant thin resin layer was etched with argon gas and oxygen gas to determine the reactive ion etching rates by these gases.

(Method of measuring reactive ion etching rates)

A glass piece was attached to the cured thin layer, and etching was performed using a reactive ion etching apparatus under the following conditions. Thereafter, the glass piece was removed, and the difference in level was measured between a portion of the thin layer that had been protected with the glass piece and a portion thereof that had been etched.

Etching rate (nm/sec) = step (nm) ÷ processing time (sec)

Reactive ion etching conditions

(Argon gas)
Etching gas: argon
Pressure: 0.5 Pa
Gas flow rate: 40 sccm
Plasma voltage: 200 W
Bias voltage: 20 W
Processing time: 200 sec
(Oxygen gas)
Etching gas: oxygen
Pressure: 0.5 Pa
Gas flow rate: 40 sccm
Plasma voltage: 200 W
Bias voltage: 20 W
Processing time: 20 sec

Table 1 sets forth the etching rates by the gases in Examples 1 to 3 and Comparative Example 1.

The resins of Examples 1 to 3 showed high argon etching resistance, and the argon etching rates thereof were lower than that in Comparative Example 1. Further, the ratios between the oxygen and argon etching rates of these resins were higher than that in Comparative Example 1. These results show that the cured layers obtained from the curable compositions for transfer materials of the invention have higher argon resistance and higher etching selectivity compared to the cured layer of Comparative Example and thus may be suitably used as resists.

[Table 1]

Table 1

| | Ex. 1 | Ex. 2 | Ex. 3 | Comp. Ex. 1 |
|---|---|---|---|---|
| $O_2$ etching rate (nm/sec) | 2.81 | 3.50 | 3.32 | 3.95 |
| Ar etching rate (nm/sec) | 0.18 | 0.18 | 0.20 | 0.31 |
| Etching selectivity ($O_2$/Ar) | 15.6 | 19.4 | 16.6 | 12.7 |

[Example 4]

A 100 ml three-necked flask was charged with 5.0 g (82.5 mmol) of urea, 38.3 g (0.33 mol) of hydroxyethyl acrylate, 9.91 g (0.33 mol) of paraformaldehyde, 15 mg (0.08 mmol) of paratoluenesulfonic acid monohydrate and 5 mg (0.04 mmol) of paramethoxyphenol. A Liebig condenser equipped with an evaporation flask was connected to the three-necked flask. The three-necked flask was then immersed in an oil bath set at 110°C, and a reaction was carried out while the mixture liquid was bubbled and stirred with dry air. With the progress of the reaction, water accumulated in the evaporation flask that was attached to the Liebig condenser connected with the three-necked flask. When the reaction was performed for 7 hours, the evaporation of water from the reaction system was confirmed to have ceased. The three-necked flask was then lifted from the oil bath, and the reaction was terminated, thereby obtaining a white turbid liquid product (d).

[Example 5]

A 300 ml three-necked flask equipped with a thermometer and a stirrer was charged with 3.59 g (15.0 mmol) of 1,1-(bisacryloyloxymethyl)ethyl isocyanate (KARENZ BEI manufactured by SHOWA DENKO K.K.), 2.12 g (15.0 mmol) of 2-acryloyloxyethyl isocyanate (KARENZ AOI manufactured by SHOWA DENKO K.K.), 10.0 mg (1.58 x $10^{-2}$ mmol) of dibutyl tin dilaurate, 5 mg (1.88 x $10^{-5}$ mmol) of 2,6-di-tert-butyl-p-cresol and 0.27 g (15.0 mmol) of water. The materials were stirred at room temperature in an air atmosphere. A white solid was gradually precipitated. After 2 hours, the reaction completed and the precipitation of the solid ceased. White solid powder (e) was thus obtained.

[Example 6]

950 parts by mass of N,N-dimethylacetamide and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white solid powder (a) from Example 1 to dissolve the same. The solution was filtered through a 0.2 μm filter. The resultant curable composition for transfer materials in a volume of 0.5 ml was dropped onto a glass substrate set in a spin coater. The glass substrate was rotated at 500 rpm for 5 seconds, 3000 rpm for 2 seconds and 5000 rpm for 20 seconds, thereby forming a thin layer on the glass substrate.

A quartz glass mold as shown in Fig. 2 in which projections and depressions were patterned (the depth of the depressions: 150 nm) along the radial direction of the mold was placed, with the patterned surface downward, on the resin-coated surface of the glass substrate with the curable composition layer. They were set in UV nanoimprinting press machine ST200 (manufactured by TOSHIBA MACHINE CO., LTD.) and pressed together, and UV ray with 365 nm wavelength and 6.5 mW/cm$^2$ intensity was applied from thereabove. The circular plate with the mold thereon was removed from the press machine, and the mold was separated. The coating layer on the glass circular plate was observed and was found to be free of defects such as pattern transfer failure or unevenness of the coating layer.

Fig. 7 shows a scanning electron micrograph (SEM) of a cross section of the transferred substrate. As shown in Fig. 7, the rectangular pattern of the mold was excellently transferred.

[Example 7]

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white solid powder (b) from Example 2 to dissolve the same. The solution was filtered through a 0.2 μm filter. The resultant curable composition for transfer materials in a volume of 0.5 ml was dropped onto a glass substrate set in a spin coater. The glass substrate was rotated at 500 rpm for 5 seconds, 3000 rpm for 2 seconds and 5000 rpm for 20 seconds, thereby forming a thin layer on the glass substrate. Subsequently, a quartz glass mold as shown in Fig. 2 in which projections and depressions were patterned (the depth of the depressions: 150 nm) along the radial direction of the mold was placed, with the patterned surface downward, on the resin-coated surface of the glass substrate with the curable composition layer. They were set in UV nanoimprinting press machine ST200 (manufactured by TOSHIBA MACHINE CO., LTD.) and pressed together, and UV ray with 365 nm wavelength and 6.5 mW/cm$^2$ intensity was applied from thereabove. The circular plate with the mold thereon was removed from the press machine, and the mold was separated. The coating layer on the glass circular plate was observed and was found to be free of defects such as pattern transfer failure or unevenness of the coating layer.

Fig. 8 shows a scanning electron micrograph (SEM) of a cross section of the transferred substrate. As shown in Fig. 8, the rectangular pattern of the mold was excellently transferred.

[Example 8]

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white solid powder (c) from Example 3 to dissolve the same. The solution was filtered through a 0.2 μm filter. The resultant curable composition for transfer materials in a volume of 0.5 ml was dropped onto a glass substrate set in a spin coater. The glass substrate was rotated at 500 rpm for 5 seconds, 3000 rpm for 2 seconds and 5000 rpm for 20 seconds, thereby forming a thin layer on the glass substrate. Subsequently, a quartz glass mold as shown in Fig. 2 in which projections and depressions were patterned (the depth of the depressions: 150 nm) along the radial direction of the mold was placed, with the patterned surface downward, on the resin-coated surface of the glass substrate with the curable composition layer. They were set in UV nanoimprinting press machine ST200 (manufactured by TOSHIBA MACHINE CO., LTD.) and pressed together, and UV ray with 365 nm wavelength and 6.5 mW/cm$^2$ intensity was applied from thereabove. The circular plate with the mold thereon was removed from the press machine, and the

mold was separated. The coating layer on the glass circular plate was observed and was found to be free of defects such as pattern transfer failure or unevenness of the coating layer.

Fig. 9 shows a scanning electron micrograph (SEM) of a cross section of the transferred substrate. As shown in Fig. 9, the rectangular pattern of the mold was excellently transferred.

[Example 9]

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white turbid liquid product (d) from Example 4 to dissolve the same. The solution was filtered through a 0.2 μm filter. The resultant curable composition for transfer materials in a volume of 0.5 ml was dropped onto a glass substrate set in a spin coater. The glass substrate was rotated at 500 rpm for 5 seconds, 3000 rpm for 2 seconds and 5000 rpm for 20 seconds, thereby forming a thin layer on the glass substrate. Subsequently, a quartz glass mold as shown in Fig. 2 in which projections and depressions were patterned (the depth of the depressions: 150 nm) along the radial direction of the mold was placed, with the patterned surface downward, on the resin-coated surface of the glass substrate with the curable composition layer. They were set in UV nanoimprinting press machine ST200 (manufactured by TOSHIBA MACHINE CO., LTD.) and pressed together, and UV ray with 365 nm wavelength and 6.5 $mW/cm^2$ intensity was applied from thereabove. The circular plate with the mold thereon was removed from the press machine, and the mold was separated. The coating layer on the glass circular plate was observed and was found to be free of defects such as pattern transfer failure or unevenness of the coating layer.

Fig. 10 shows a scanning electron micrograph (SEM) of a cross section of the transferred substrate. As shown in Fig. 10, the rectangular pattern of the mold was excellently transferred.

[Example 10]

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of the white solid powder (e) from Example 5 to dissolve the same. The solution was filtered through a 0.2 μm filter. The resultant curable composition for transfer materials in a volume of 0.5 ml was dropped onto a glass substrate set in a spin coater. The glass substrate was rotated at 500 rpm for 5 seconds, 3000 rpm for 2 seconds and 5000 rpm for 20 seconds, thereby forming a thin layer on the glass substrate. Subsequently, a quartz glass mold as shown in Fig. 2 in which projections and depressions were patterned (the depth of the depressions: 150 nm) along the radial direction of the mold was placed, with the patterned surface downward, on the resin-coated surface of the glass substrate with the curable composition layer. They were set in UV nanoimprinting press machine ST200 (manufactured by TOSHIBA MACHINE CO., LTD.) and pressed together, and UV ray with 365 nm wavelength and 6.5 $_{M}W/_{CM}{}^2$ intensity was applied from thereabove. The circular plate with the mold thereon was removed from the press machine, and the mold was separated. The coating layer on the glass circular plate was observed and was found to be free of defects such as pattern transfer failure or unevenness of the coating layer.

Fig. 11 shows a scanning electron micrograph (SEM) of a cross section of the transferred substrate. As shown in Fig. 11, the rectangular pattern of the mold was excellently transferred.

[Comparative Example 2]

950 parts by mass of propylene glycol monomethyl ether acetate and 1.5 parts by mass of 2-benzyl-2-dimethylamino-1-(4-morpholinophenyl)-1-butanone (IRGACURE 369 manufactured by Ciba Japan K.K.) were added to 50 parts by mass of EBECRYL 8807 (manufactured by DAICEL-CYTEC COMPANY LTD., an aliphatic urethane acrylate compound) to dissolve the same. The solution was filtered through a 0.2 μm filter. The resultant curable composition for transfer materials in a volume of 0.5 ml was dropped onto a glass substrate set in a spin coater. The glass substrate was rotated at 500 rpm for 5 seconds, 3000 rpm for 2 seconds and 5000 rpm for 20 seconds, thereby forming a thin layer on the glass substrate. Subsequently, a quartz glass mold as shown in Fig. 2 in which projections and depressions were patterned (the depth of the depressions: 150 nm) along the radial direction of the mold was placed, with the patterned surface downward, on the resin-coated surface of the glass substrate with the curable composition layer. They were set in UV nanoimprinting press machine ST200 (manufactured by TOSHIBA MACHINE CO., LTD.) and pressed together, and UV ray with 365 nm wavelength and 6.5 $mW/cm^2$ intensity was applied from thereabove. The circular plate with the mold thereon was removed from the press machine, and the mold was separated. The coating layer on the glass circular plate was observed, but defects such as pattern transfer failure or unevenness of the coating layer were not found.

Fig. 12 shows a scanning electron micrograph (SEM) of a cross section of the transferred substrate. As shown

in Fig. 12, the pattern transferred was not substantially rectangular.

Reference Signs List

12 MOLD

14 COATING LAYER OF CURABLE COMPOSITION OF THE INVENTION

16 SUBSTRATE

18 ENERGY RAY AND/OR HEAT

20 REACTIVE ION ETCHING

22 BASE

24 MAGNETIC LAYER

26 FINE PATTERN PRODUCED FROM CURABLE COMPOSITION

28 ION MILLING

30 REACTIVE GAS

32 DEMAGNETIZED LAYER

A ENLARGED VIEW

## Claims

1. A curable composition for transfer materials which comprises a compound (A) having at least one skeleton selected from the group consisting of Formulae (1) to (3) below, and a (meth)acryloyl group:

[Chem. 1]

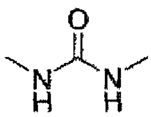

(1)

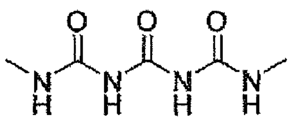

(2)

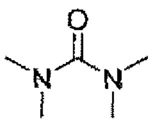

(3)

2. The curable composition for transfer materials according to claim 1, wherein the compound (A) has 1 to 3 skeletons

of Formula (1) in the molecule.

**3.** The curable composition for transfer materials according to claim 1 or 2, wherein the compound (A) is at least one (meth)acryloyl group-containing urea compound selected from the group consisting of Formulae (4) and (5) below:

[Chem. 2]

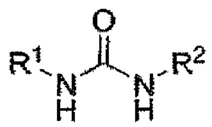

(4)

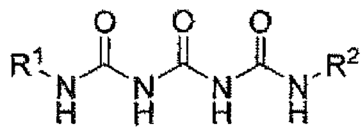

(5)

wherein $R^1$ and $R^2$ in Formulae (4) and (5) are each independently represented by any of Formulae (i) to (iii) below:

[Chem. 3]

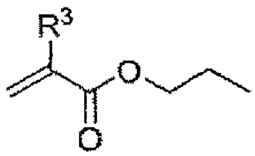

(i)

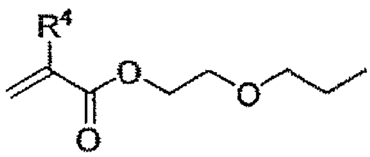

(ii)

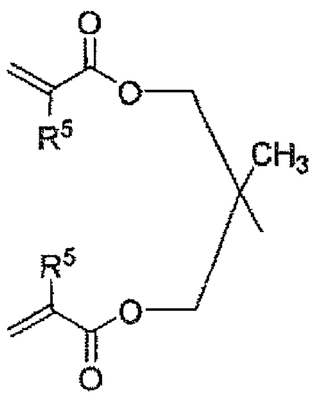

(iii)

wherein $R^3$ to $R^5$ in Formulae (i) to (iii) are each independently a hydrogen atom or a methyl group.

**4.** The curable composition for transfer materials according to any one of claims 1 to 3, wherein the compound (A) is a compound obtained by reacting water and at least one (meth)acryloyl group-containing isocyanate selected from the group consisting of Formulae (iv) to (vi) below:

[Chem. 4]

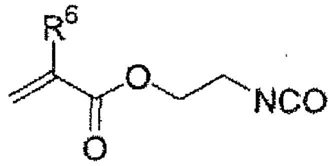

(iv)

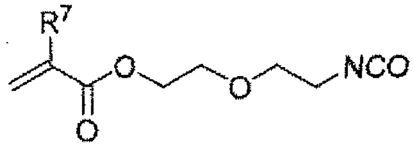

(v)

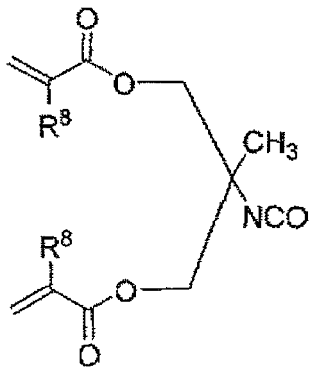

(vi)

wherein $R^6$, $R^7$ and $R^8$ in Formulae (iv) to (vi) are each independently a hydrogen atom or a methyl group.

**5.** The curable composition for transfer materials according to any one of claims 1 to 3, wherein the compound (A) is a compound obtained by reacting urea and at least one (meth)acryloyl group-containing isocyanate selected from the group consisting of Formulae (iv) to (vi) below:

[Chem. 5]

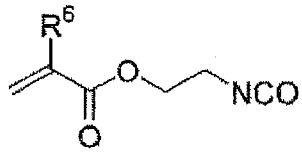

(i v)

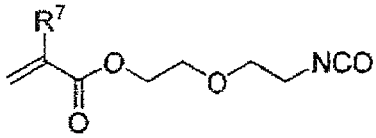

(v)

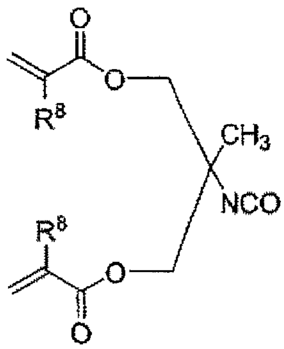

(v i)

wherein $R^6$, $R^7$ and $R^8$ in Formulae (iv) to (vi) are each independently a hydrogen atom or a methyl group.

**6.** The curable composition for transfer materials according to claim 1 or 2, wherein the compound (A) is a compound obtained by reacting urea, a compound of Formula (vii) below having a hydroxyl group and a (meth)acryloyl group, and paraformaldehyde;

[Chem. 6]

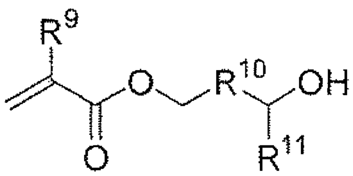

(v i i)

wherein in Formula (vii), $R^9$ is a hydrogen atom or a methyl group, $R^{10}$ is a single bond or a C1-6 saturated hydrocarbon group, and $R^{11}$ is a hydrogen atom or a methyl group.

**7.** The curable composition for transfer materials according to claim 6, wherein the compound of Formula (vii) is at least one selected from 2-hydroxyethyl (meth)acrylate, 2-hydroxypropyl (meth)acrylate, 3-hydroxybutyl (meth)acrylate, 4-hydroxybutyl (meth)acrylate and 1,4-cyclohexanedimethanol mono(meth)acrylate.

**8.** The curable composition for transfer materials according to any one of claims 1 to 7, which further comprises a radically polymerizable compound other than the compound (A) .

**9.** A pattern forming process comprising:

a step of applying the curable composition for transfer materials described in any one of claims 1 to 8 to a substrate to form a coating layer;
a step of pressing a mold having a pattern of projections and depressions on its surface, into the coating layer;
a step of heating the coating layer while keeping the mold pressed in the coating layer, thereby to cure the coating layer; and
a step of separating the mold from the cured coating layer.

**10.** A pattern forming process comprising:

a step of applying the curable composition for transfer materials described in any one of claims 1 to 8 to a substrate to form a coating layer;
a step of pressing a mold having a pattern of projections and depressions on its surface, into the coating layer;
a step of applying an active energy ray to the coating layer while keeping the mold pressed in the coating layer, thereby to cure the coating layer; and
a step of separating the mold from the cured coating layer.

**11.** The pattern forming process according to claim 10, wherein the mold is formed of a material that transmits active energy rays, and the active energy ray is applied to the coating layer through the mold.

**12.** The pattern forming process according to claim 10, wherein the substrate is formed of a material that transmits active energy rays, and the active energy ray is applied to the coating layer through the substrate.

**13.** A process for manufacturing magnetic recording media, comprising:

providing a substrate comprising a base and a magnetic layer thereon and forming a pattern on the magnetic layer by the process described in any one of claims 9 to 12; and
removing a part of the magnetic layer or demagnetizing a part of the magnetic layer using the pattern as a resist.

**14.** A magnetic recording medium obtainable by the process described in claim 13.

**15.** A magnetic recording and reading apparatus comprising the magnetic recording medium described in claim 14.

**16.** A (meth)acryloyl group-containing urea compound represented by Formula (6) below:

[Chem. 7]

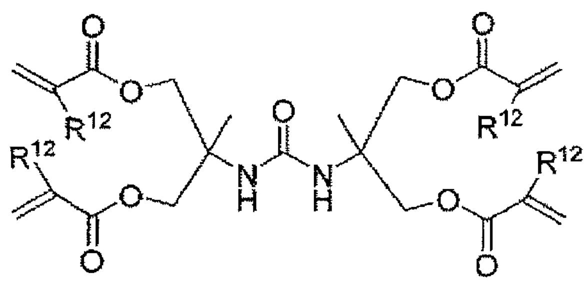

(6)

wherein $R^{12}$ in Formula (6) is a hydrogen atom or a methyl group.

[Fig. 1]

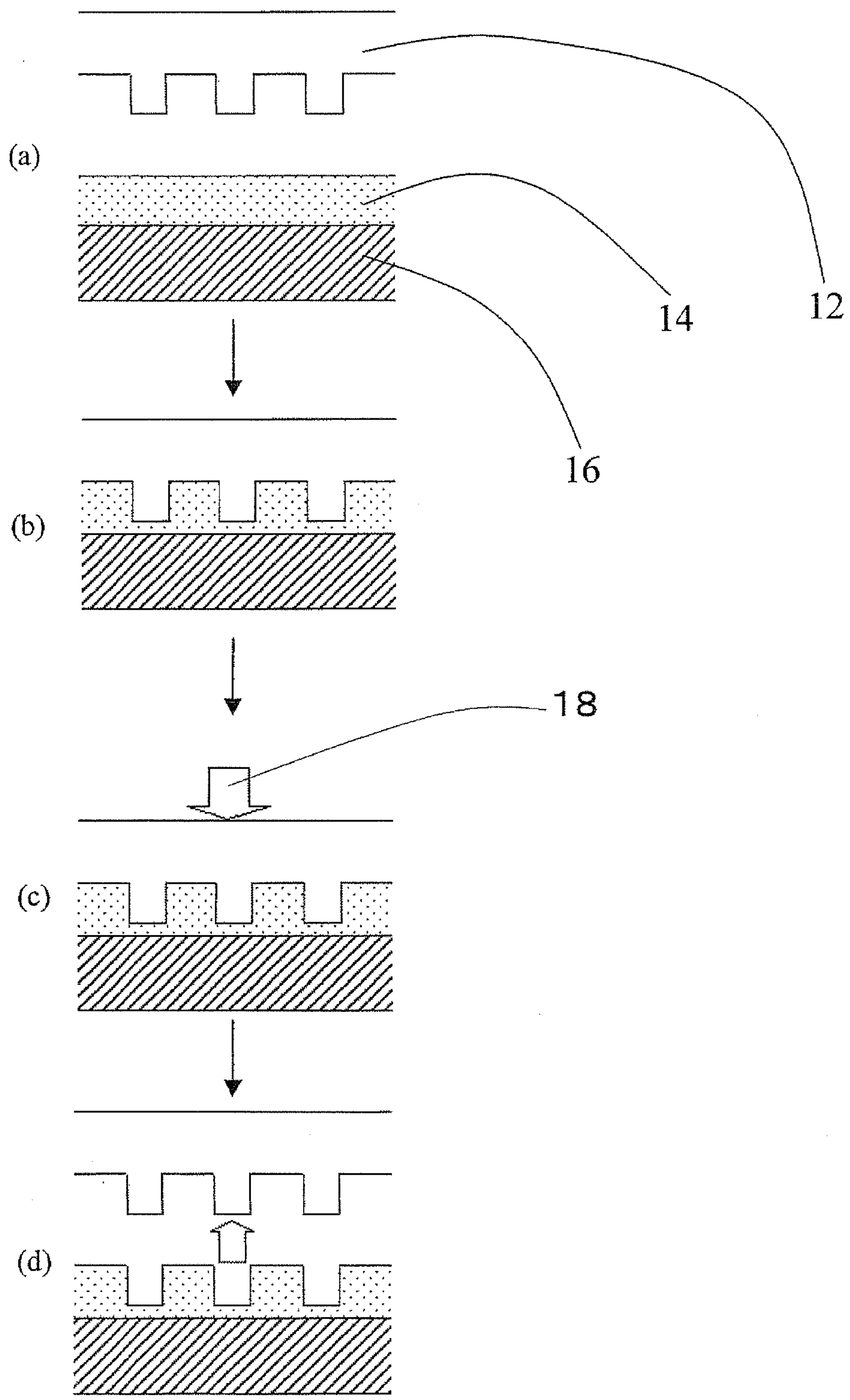

[Fig. 2]

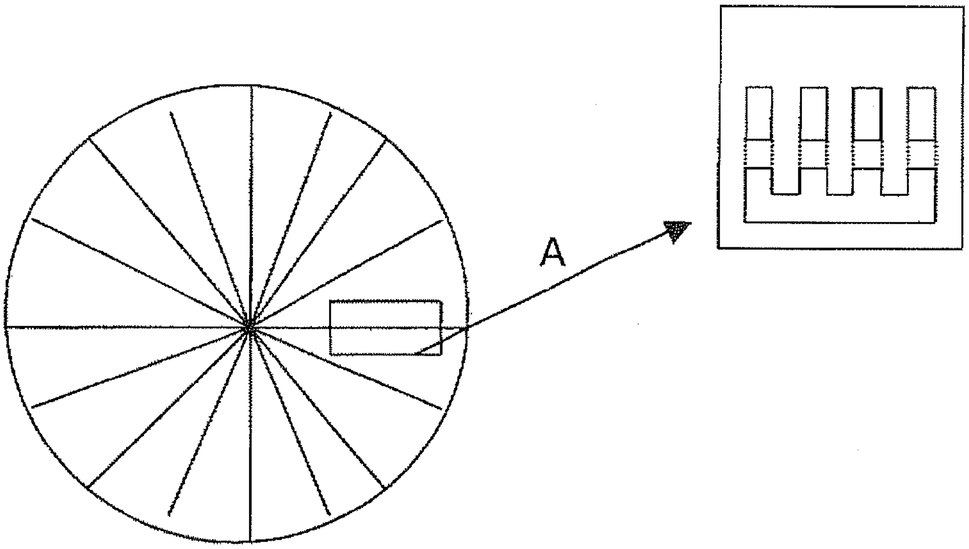


[Fig. 3]

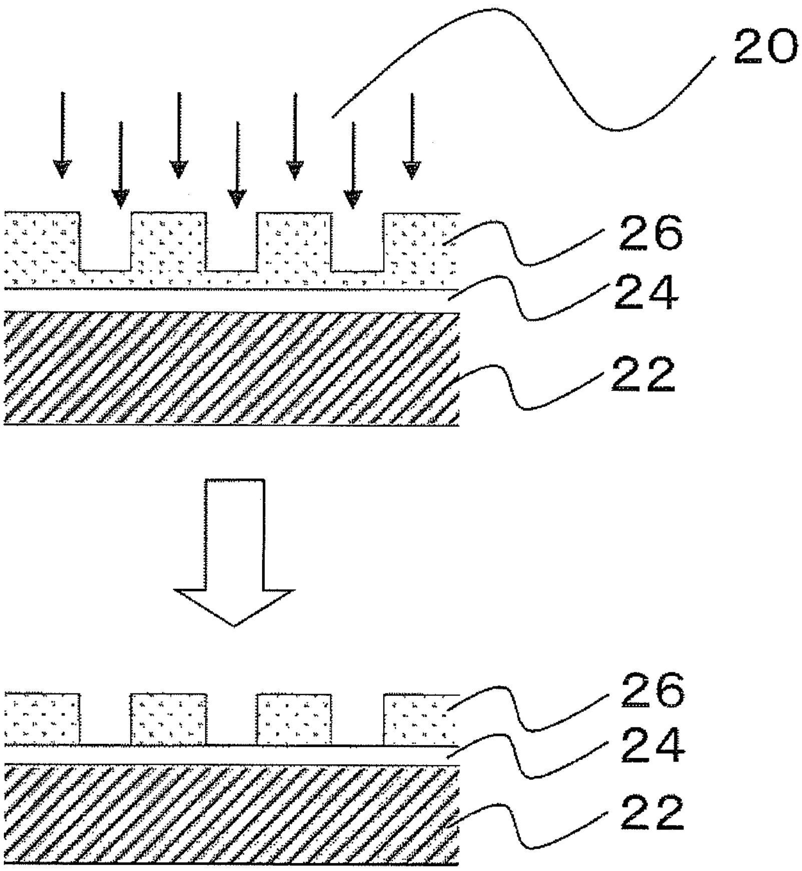

[Fig. 4]

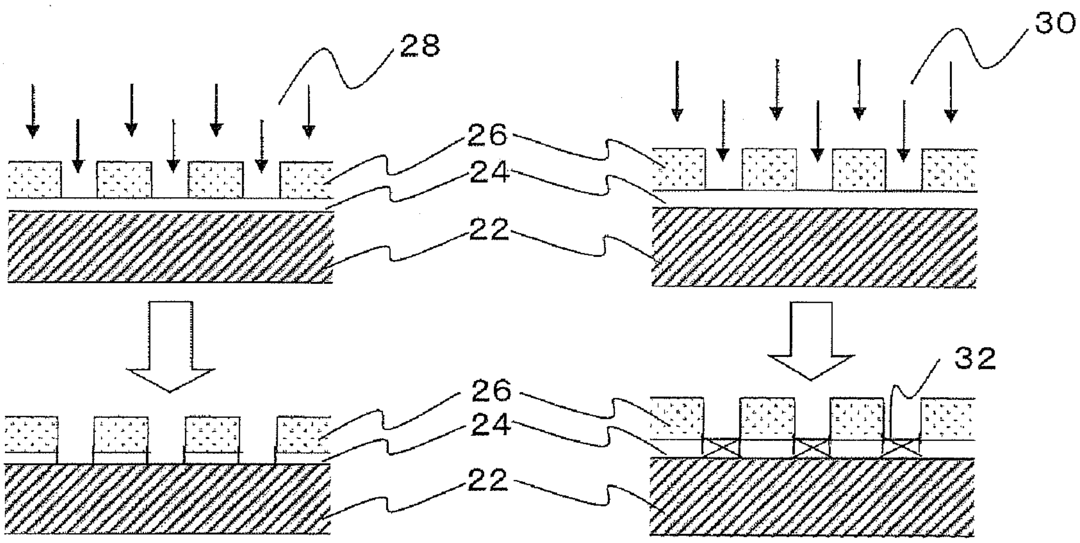

[Fig. 5]

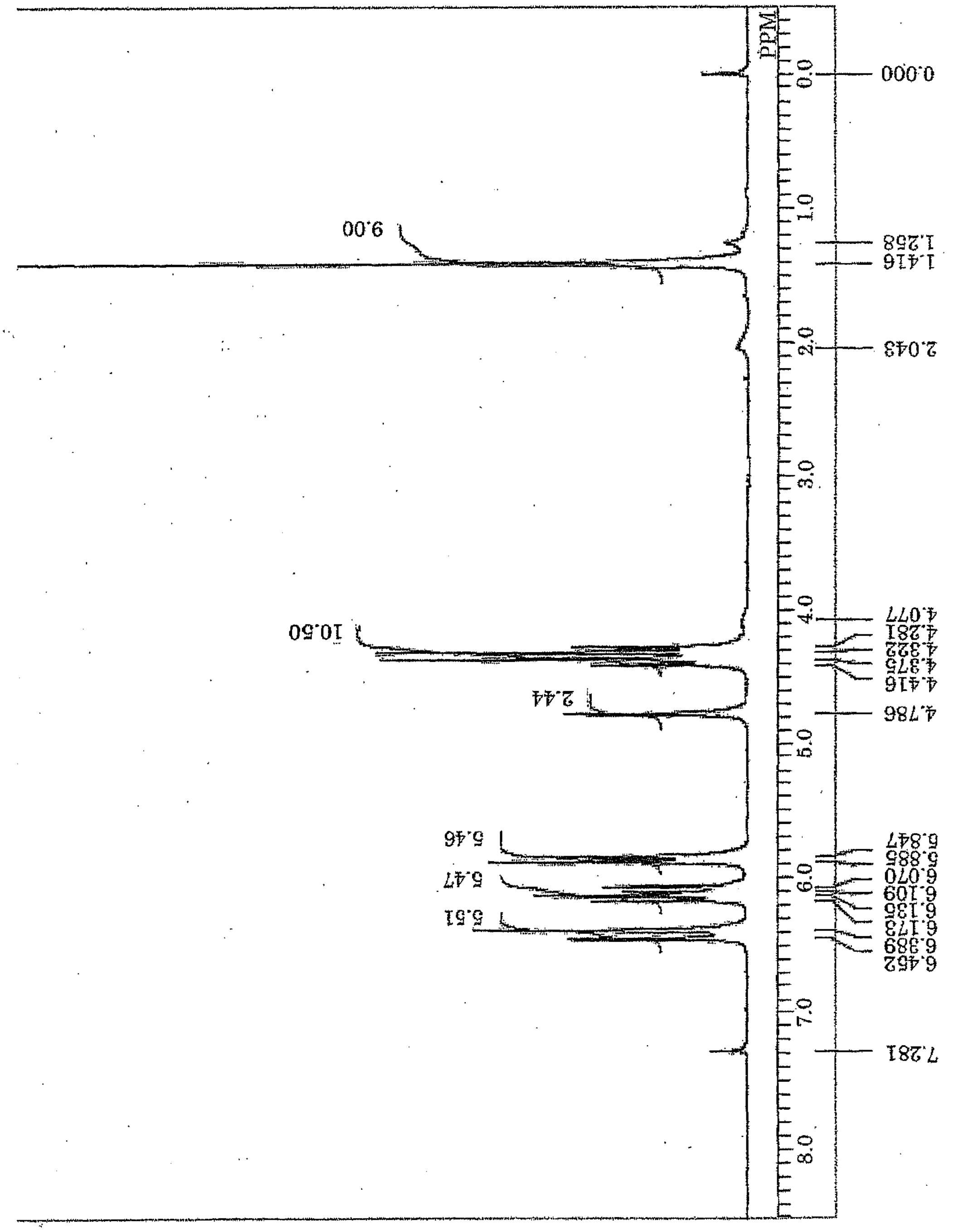
PPM
0.0
1.0
2.0
3.0
4.0
5.0
6.0
7.0
8.0
0.000
1.258
1.416
2.048
4.077
4.281
4.322
4.375
4.416
4.786
5.847
5.885
6.070
6.109
6.136
6.173
6.389
6.452
7.281
9.00
10.50
2.44
5.46
5.47
5.51

[Fig. 6]

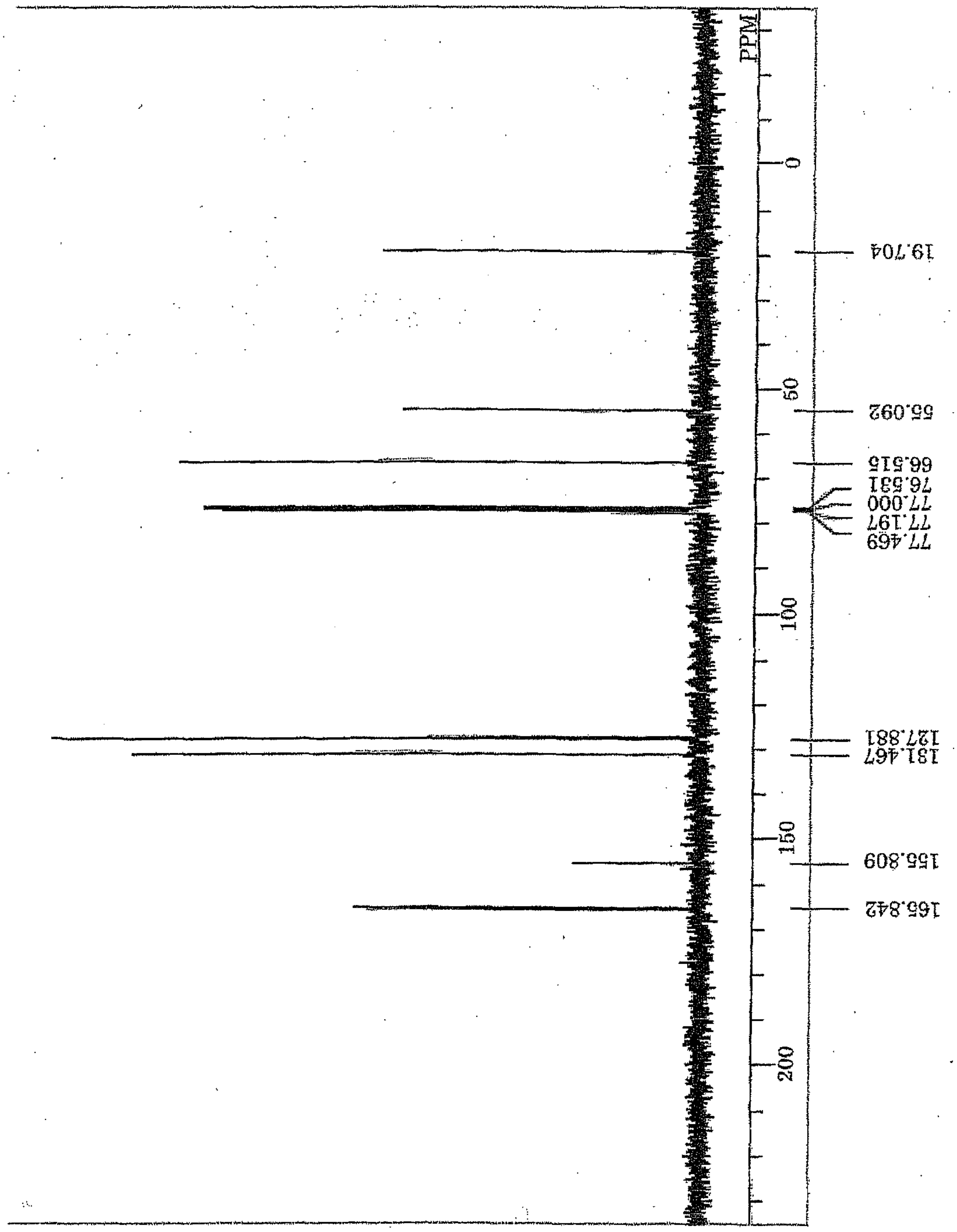
PPM
0
50
100
150
200
19.704
55.092
66.515
76.531
77.000
77.197
77.469
127.881
131.467
155.809
165.842

[Fig. 7]

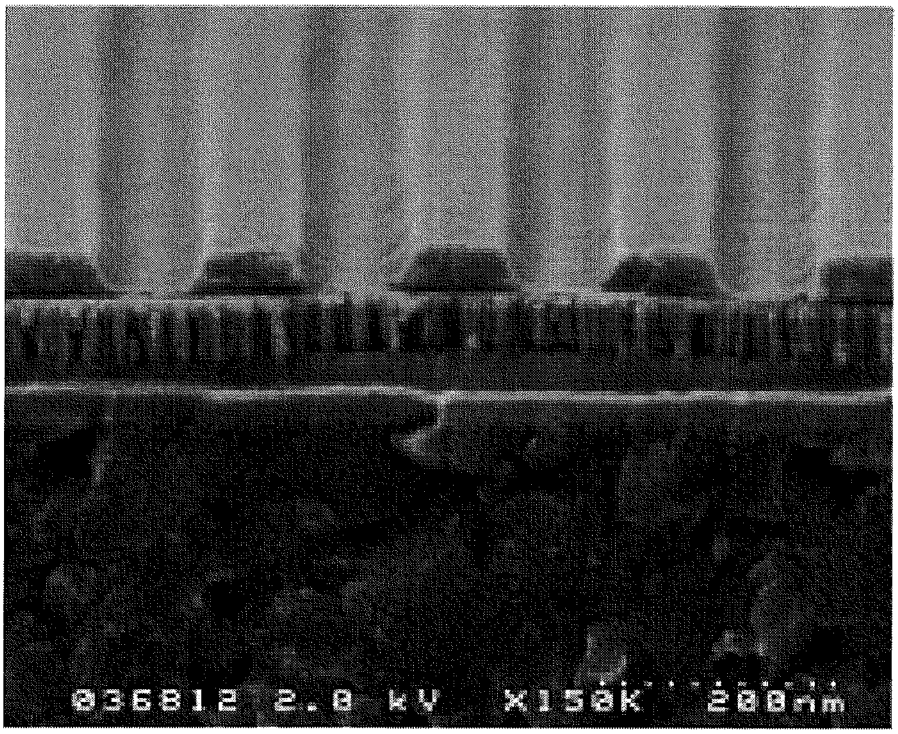


[Fig. 8]

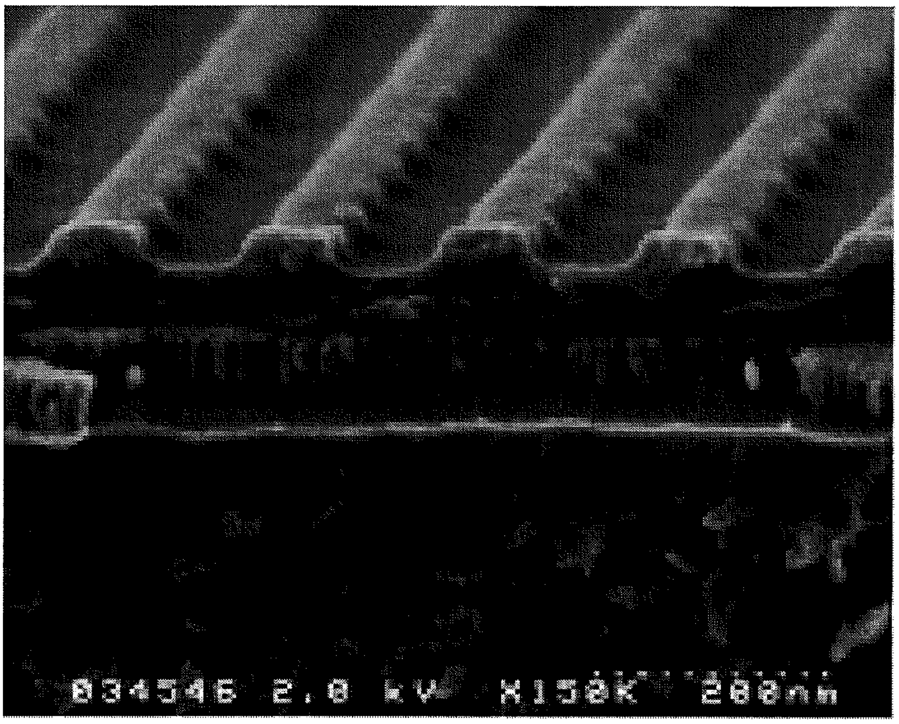

[Fig. 9]

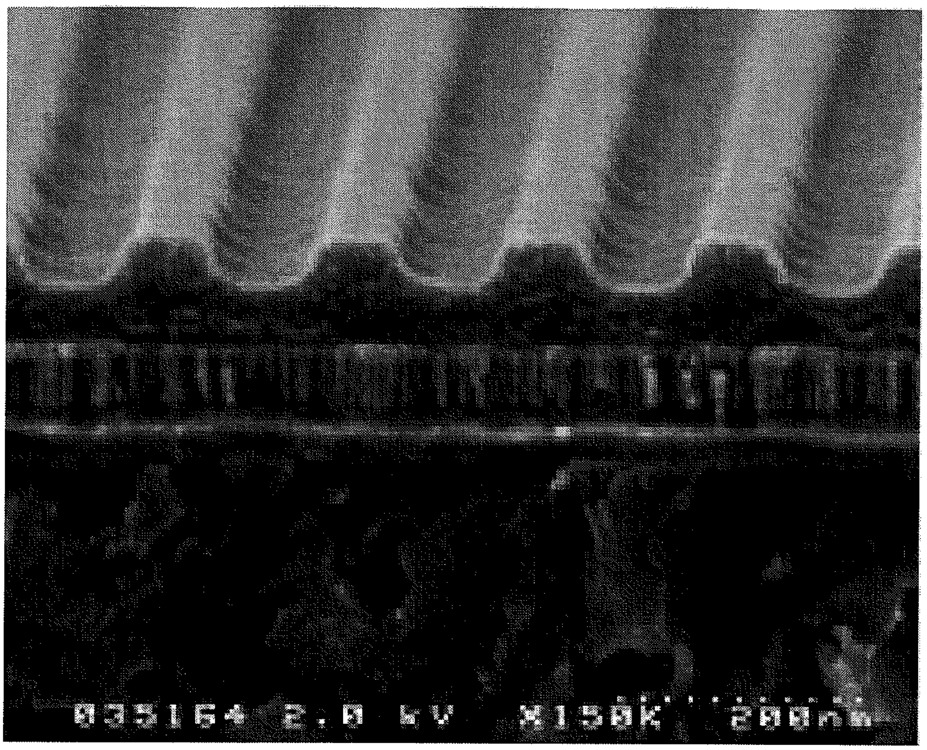


[Fig. 10]

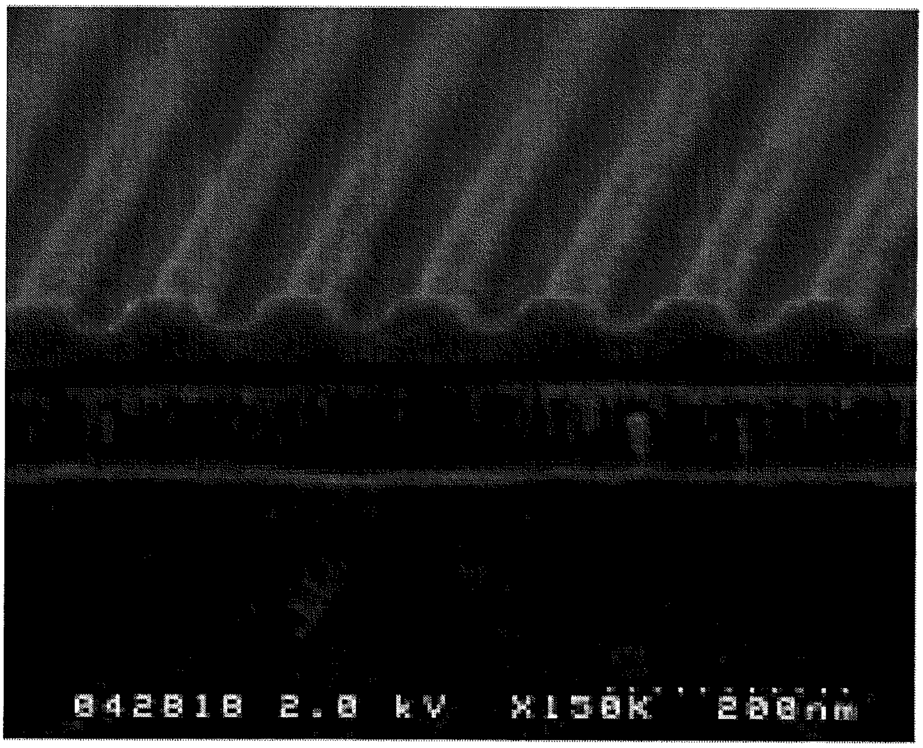

[Fig. 11]

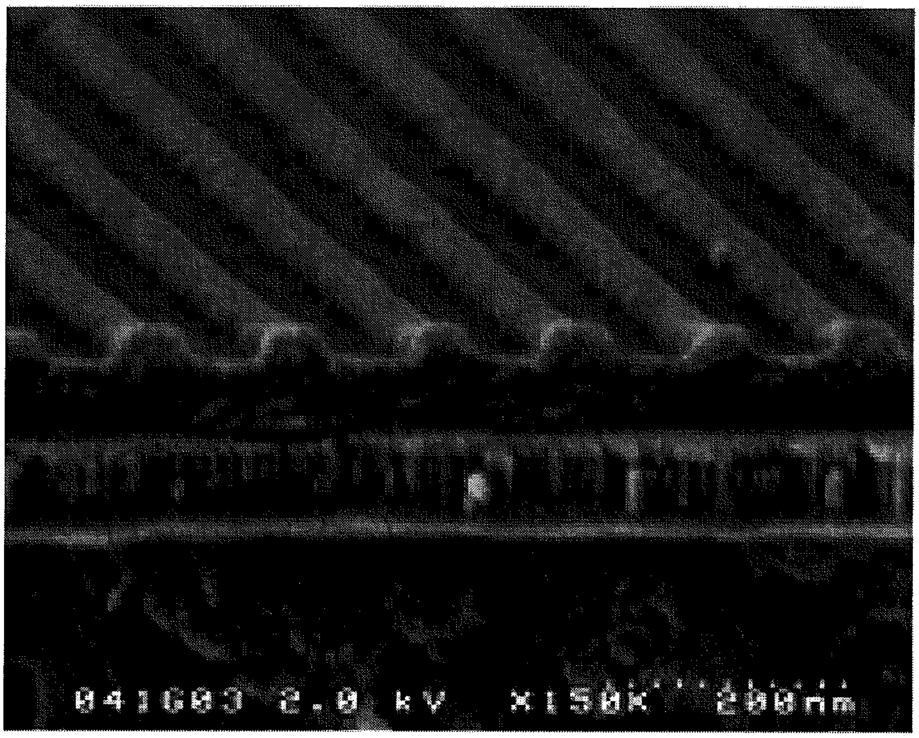


[Fig. 12]

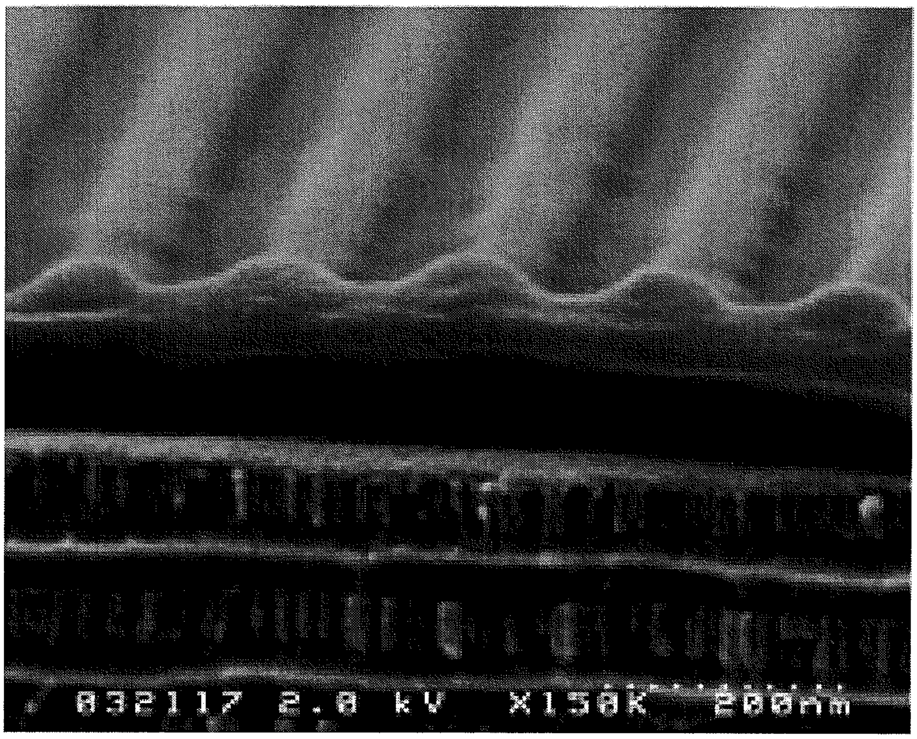

**INTERNATIONAL SEARCH REPORT**

International application No.
PCT/JP2010/050936

A. CLASSIFICATION OF SUBJECT MATTER
*H01L21/027*(2006.01)i, *B29C59/02*(2006.01)i, *C07C275/10*(2006.01)i, *G11B5/65*(2006.01)i, *G11B5/84*(2006.01)i, *G11B5/855*(2006.01)i, *B29K61/20*(2006.01)n

According to International Patent Classification (IPC) or to both national classification and IPC

B. FIELDS SEARCHED

Minimum documentation searched (classification system followed by classification symbols)
H01L21/027, B29C53/00-53/84, 57/00-59/18, G03F7/004-7/06, 7/075-7/115, 7/16-7/18, C07C273/00-275/70

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2008-105414 A (FUJIFILM CORP.),<br>08 May 2008 (08.05.2008),<br>entire text (particularly, claims 1, 4; paragraph [0192])<br>& CN 101154042 A & KR 10-2008-0028786 A | 14,15<br>1-15 |
| Y | JP 2008-242093 A (FUJIFILM CORP.),<br>09 October 2008 (09.10.2008),<br>entire text (particularly, paragraphs [0001], [0017], [0022])<br>& US 2008/0241744 A1 & EP 1975707 A1 | 1-15 |
| Y | JP 05-100424 A (HITACHI CHEMICAL CO., LTD.),<br>23 April 1993 (23.04.1993),<br>entire text (particularly, claim 1; paragraph [0025])<br>(Family: none) | 1-16 |

☒ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 April, 2010 (26.04.10) | 18 May, 2010 (18.05.10) |
| Name and mailing address of the ISA/<br>Japanese Patent Office<br>Facsimile No. | Authorized officer<br><br>Telephone No. |

Form PCT/ISA/210 (second sheet) (July 2009)

INTERNATIONAL SEARCH REPORT

International application No.
PCT/JP2010/050936

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2006-063318 A (TOKYO OHKA KOGYO CO., LTD.),<br>09 March 2006 (09.03.2006),<br>entire text (particularly, claim 3)<br>& WO 2006/011420 A1 | 5 |
| Y | JP 50-000648 B1 (MITSUI TOHATSU CHEMICAL CO.),<br>10 January 1975 (10.01.1975),<br>entire text<br>(Family: none) | 5 |
| Y | JP 2007-055993 A (SHOWA DENKO KABUSHIKI KAISHA),<br>08 March 2007 (08.03.2007),<br>entire text (particularly, claim 6; paragraph [0036])<br>& US 2008/0132597 A1 & EP 1812381 A1<br>& WO 2006/049264 A1 & KR 10-2007-0085683 A<br>& CN 101084186 A | 16 |
| A | JP 2002-012665 A (ASAHI KASEI CORP.),<br>15 January 2002 (15.01.2002),<br>entire text<br>(Family: none) | 1-16 |
| A | JP 2003-277723 A (NITTO DENKO CORP.),<br>02 October 2003 (02.10.2003),<br>entire text<br>(Family: none) | 1-16 |

Form PCT/ISA/210 (continuation of second sheet) (July 2009)

**INTERNATIONAL SEARCH REPORT**

International application No.
PCT/JP2010/050936

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The invention in claim 1 and the invention in claim 16 have a common technical feature in such a point that both inventions relate to urea compound containing Acryloyl group. However, the above-said technical feature cannot be a special technical feature, since the feature does not make contribution over the prior art in the light of JP 2008-242093 A (FUJIFILM CORP.), 9 October 2008 (09.10.2008) and JP 05-100424 A (HITACHI CHEMICAL CO.,LTD.), 23 April 1993 (23.04.1993).

(continued to extra sheet)

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**

International application No.
PCT/JP2010/050936

Continuation of Box No.III of continuation of first sheet(2)

Furthermore, there is no other same or corresponding special technical feature between said inventions.

The following two inventions (invention groups) are involved in claims.

(Invention 1) the invention in claims 1 - 15

(Invention 2) the invention in claim 16

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003100609 A
- JP 2005277280 A
- JP 2007072374 A
- JP 2003287889 A
- JP 2007273067 A